# FASCICULE DE BREVET EUROPEEN

(11) **EP 0 528 932 B1**
(45) Date de publication et mention de la délivrance du brevet: **13.03.1996**
(21) Numéro de dépôt: 91909638.8
(22) Date de dépôt: 18.04.1991
(51) Int. Cl.: C07K 14/47, C12N 15/12, A61K 49/00, G01N 33/577

(54) **PROTEINE ASSOCIEE A LA PANCREATITE AIGUE, MOYENS POUR LE DIAGNOSTIC DE LA PANCREATITE AIGUE**
MIT AKUTER PANKREATITIS ASSOZIIERTES PROTEIN, MITTEL ZUR DIAGNOSE VON AKUTER PANKREATITIS
ACUTE PANCREATITIS ASSOCIATED PROTEIN AND MEANS FOR THE DIAGNOSIS OF ACUTE PANCREATITIS

(30) Priorité: 20.04.1990 FR 9005062
(43) Date de publication de la demande: 03.03.1993
(73) Titulaire: INSTITUT NATIONAL DE LA SANTE ET DE LA RECHERCHE MEDICALE (INSERM), F-75654 Paris Cédex 13 (FR)
(72) Inventeur: IOVANNA, Juan-Lucio 151, traverse de la Gouffonne, F-13009 Marseille (FR); KEIM, Volker, D-6805 Heddesheim (DE); DAGORN, Jean-Charles 77, bd du Redon, F-13009 Marseille (FR)
(74) Mandataire: Desaix, Anne
(86) Numéro de dépôt international: FR9100323
(87) Numéro de publication internationale: WO9116428

(56) Documents cités:
- GASTROENTEROLOGY, Vol. 100, No. 3, March 1991, pages 755-782; KEIM, V. et al
- GASTROENTROLOGY, Vol. 98, No. 5pt2, May 1990, page A220; IOVANNA, J. et al.
- DIGESTION, Vol. 31, No. 3, 25 September 1985, page 191; KEIM, V. et al
- DIGESTIVE DISEASES AND SCIENCES, Vol. 30, No. 10, October 1985, page 977; KEIM, V. & ROHR, G.
- DIGESTIVE DISEASES AND SCIENCES, Vol. 30, No. 10, October 1985, page 988; ROHR, G. et al
- DIGESTION, Vol. 29, 1984, pages 242-249; KEIM, V. et al
- CLIN. PHYSIOL. BIOCHEM, Vol. 4, 1986, pages 136-142; KEIM, V.

## Description

La présente invention concerne des protéines associées à la pancréatite aiguë et des moyens pour le diagnostic de cette maladie.

La pancréatite aiguë est une affection inflammatoire du pancréas qui, sur le plan anatomopathologique, va de la simple forme oedémateuse à la nécrose hémorragique totale de la glande. La pancréatite nécro-hémorragique est une affection très grave, puisque l'estimation de sa mortalité varie de 30 % à 70 % selon les auteurs. Il est dans certains cas, très difficile d'établir avec certitude le diagnostic de pancréatite aiguë (Sarner, M. et al, Gastroenterol. (1984), 13 : 865-870). Ce diagnostic repose avant tout sur l'examen clinique (douleur abdominale aiguë) sur le dosage d'un certain nombre de substances dans le plasma ou dans le liquide péritonéal (Bradley, J. et al, Br. J. Surg. (1981), 68 : 245-246 ; et Dubick, M. et al, Dig. Dis. Sci. (1987), 32 : 305-312). Parmi les dosages employés, on trouve ceux de l'amylase, de la lipase, de la trypsine, de l'élastase, de la ribonucléase, de la phospholipase A2, de l'α2 macroglobuline, du calcium, de la LDH, des inhibiteurs de protéases et d'autres encore. Aucun d'entre eux ne s'avère pourtant spécifique, pratique, ni surtout discriminatif. On se contente donc en général de doser l'amylasémie. Récemment, l'échotomographie et la tomodensitométrie ont paru, dans certains cas, pouvoir faciliter le diagnostic de la pancréatite sans que le progrès ait été décisif (Silverstein, W. et al,Am.J. Roentgenol, (1981), 137 : 497-502).

Keim et al ont publié en 1984 (Digestion, (1984);, 29 : 242-249), des résultats sur les conséquences d'une cannulation du canal pancréatique et de l'induction d'une pancréatite sur la composition protéique du suc pancréatique de rat, cet animal étant utilisé comme modèle expérimental. Après l'opération de cannulation (1 à 2 jours après), les auteurs ont observé une chute du taux d'amylase dans le suc pancréatique, puis 3 à 4 jours après l'opération, un retour au niveau normal d'amylase.

Une séparation par électrophorèse en gel de polyacrylamide (PAGE) des protéines du suc pancréatique pendant cette période de rémission, montrait une bande protéique supplémentaire, apparemment au plus tôt 12 heures après l'opération, et au plus tard, 3 à 4 jours après l'opération. Cette bande protéique n'existait pas chez le rat contrôle non traité. Cette protéine secrétoire a été appelée PAP ("pancréatitis associated protein").

Par la suite, Keim et al ont effectué des mesures de la quantité de PAP présente dans le tissu pancréatique du rat après induction d'une pancréatite, au moyen de tests de détection de la fixation du complément.

Ces tests n'ont cependant pas permis jusqu'à présent de mettre en évidence l'existence de la PAP dans le sérum du rat chez lequel une pancréatite a été induite.

Les moyens proposés jusqu'à présent dans l'art antérieur n'avaient ainsi pas pu conduire à une identification suffisante de la protéine PAP chez le rat pour que le problème de l'intérêt d'une investigation chez l'homme, afin de rechercher si une telle protéine pouvait être détectée, soit posé.

De plus, les résultats disponibles jusqu'à présent, n'auraient pas permis d'envisager l'intérêt de la PAP pour réaliser un diagnostic d'une pancréatite.

Les inventeurs ont constaté que des anticorps polyclonaux de rat, reconnaissant la protéine PAP de rat, ne reconnaissaient pas de façon significative une protéine du sérum humain.

Après cette constatation, les inventeurs ont donc recherché une identification plus adéquate de la protéine PAP de rat, en vue de définir des outils d'investigation chez l'homme : les inventeurs ont maintenant clairement identifié la protéine PAP chez le rat et ils ont établi sa séquence en acides aminés. A partir de ces connaissances, ils ont élaboré des moyens qui pourraient permettre de détecter et d'identifier s'il existe une protéine correspondant à la PAP du rat, chez l'homme.

Le clonage et le séquençage de l'ARN messager PAP à partir partir d'une bibliothèque cDNA pancréatique de rat a aussi permis de démontrer sans ambigüité que la PAP est bien synthétisée par le pancréas. Les inventeurs ont aussi montré que la protéine était très faiblement exprimée en l'absence d'inflammation pancréatique et fortement exprimée au cours de la pancréatite.

Compte tenu de la fragilité des malades atteints de pancréatite, il n'était pas concevable d'envisager de recueillir le suc pancréatique de ces malades, pour y rechercher la présence éventuelle d'une protéine PAP humaine (PAP-H).

Les inventeurs ont criblé une banque de cDNA pancréatique humaine, à l'aide d'un clone cDNA préalablement obtenu et correspondant à la PAP du rat.

Les inventeurs sont parvenus à isoler différents clones contenant des fragments d'ADNc susceptibles de s'hybrider avec l'ADNc de la PAP de rat, ainsi qu'il est décrit ci-dessous.

La présente invention concerne donc des fragments d'ADNc capables de coder pour la protéine PAP de rat, ainsi que des fragments d'ADNc susceptibles de coder pour les protéines de la famille de la PAP humaine. L'invention vise aussi les protéines codées par ces fragments d'ADNc.

Elle se rapporte aussi à des vecteurs modifiés par intégration des susdits fragments d'ADNc, notamment pour l'expression de ces fragments.

L'invention vise aussi des anticorps polyclonaux ou monoclonaux dirigés contre la protéine PAP notamment la PAP humaine, ainsi que leur utilisation dans des procédés faisant appel à l'imagerie médicale, ou comme moyen pour le diagnostic de la pancréatite aiguë dans des kits et des procédés de diagnostic in vitro.

Une première famille de fragments d'ADN selon l'invention comprend donc les fragments d'ADNc codant pour la PAP de rat.

Appartiennent à cette famille, des fragments d'ADNc caractérisés en ce qu'ils répondent à la séquence nucléotidique S1 suivante codant pour la protéine PAP de rat, à une partie ou à une variante de cette séquence dès lors que cette partie ou variante code pour une protéine ou un peptide reconnu par des anticorps dirigés contre la protéine PAP de rat, ou qu'elle hybride avec la séquence S1 dans une solution d'hybridation contenant 6 x SSC, 5 x Denhart, 0,5 % SDS, 10 mM EDTA, 200 µg ADN de sperme de saumon, pendant 18 heures à 68°C et après un rinçage dans les conditions suivantes : 6 x SSC, O,1 % SDS, 2 fois 15 minutes à 65°.

Séquence S1 :

10 20 30 40 50 60
AAAACCATCC AAATCGCCCG CAAGACAGCT AAGGAGGAGC AGAAAGATGA TGAGAGTTAA
70 80 90 100 110 120
TATGTTGCAT CGCTTGGCCT TCCCAGTCAT GTCCTGGATG CTGCTCTCCT GCCTGATGCT
130 140 150 160 170 180
CTTATCACAG GTGCAAGGAG AAGACTCTCC GAAGAAAATA CCCTCTGCAC GCATTAGTTG
190 200 210 220 230 240
CCCCAAAGGC TCCCAGGCAT ATGGCTCCTA CTGCTATGCC CTGTTTCAGA TACCACAGAC
250 260 270 280 290 300
CTGGTTTGAT GCAGAACTGG CCTGCCAGAA GAGACCTGAA GGACACCTTG TATCTGTGCT
310 320 330 340 350 360
CAATGTAGCT GAAGCTTCAT TCTTGGCATC CATGGTCAAG AACACTGGAA ACAGCTACCA
370 380 390 400 410 420
ATATACCTGG ATTGGACTCC ATGACCCCAC TCTTGGTGGA GAACCCAATG GAGGTGGATG
430 440 450 460 470 480
GGAGTGGAGT AACAATGACA TAATGAATTA TGTCAACTGG GAGAGGAACC CATCTACTGC
490 500 510 520 530 540
CTTAGACCGC GGATTCTGTG GCAGCTTGTC AAGATCTTCT GGATTTCTAA GATGGAGAGA
550 560 570 580 590 600
TACCACATGT GAAGTTGAAG TTGCCCTACG TCTGCAAATT TACAGGTTAA AATTACCAGA
610 620 630 640 650 660
CAGCAAACAG CTTTAGTTTG TCCTGAAGCA CATCCTGTCA AGGGGCAAAA TATGAAGACT
670 680 690 700 710 720
TGCGTAGAAA AAGTGTATTC TATCTACAGT CCATATTGGA GCTCTAATCA TTCTTTAGCC
730 740 750 760 770 780
AATTTTGTAT AAGTTGTGTC CTCATGTCTT GGAAAGCAGT AATAAACCTC AGTCTCTCTT
790 800 810 820 830 840
CGAAAAAAAA AAA

Il est précisé que les abréviations ci-dessus ont les significations suivantes : 1 x SSC = 150 mM NaCl, 15 mM citrate de sodium ; 50 x Denhart = 5g Ficoll, 5g polyvinylpyrrolidone, 5g sérum albumine bovine dans 500 ml d'eau ; SDS : dodecylsulfate de sodium ; EDTA ; ethylène diamine tétraacétate de sodium.

Des anticorps polyclonaux dirigés contre la PAP de rat sont produits selon les techniques classiques ; de tels anticorps ont par exemple été décrits par Keim et al (Clin. Physiol. Biochem., 4 : 136-142 (1986)).

Un fragment d'ADNc appartenant à la première famille d'acides nucléiques de l'invention, est encore caractérisé en ce qu'il code pour une protéine répondant à une des séquences d'acides aminés A1 (séquence d'acides aminés de la protéine comprenant le peptide signal) ou A2 (séquence d'acides aminés de la protéine mature) suivantes ou pour une séquence d'acides aminés ayant une homologie de 40 à 80 % de préférence de 50 à 60 % avec au moins un enchaînement d'environ 25 acides aminés des séquences A1 ou A2.

Séquence A1 :

MetLeuHisArgLeuAlaPheProValMetSerTrpMetLeuLeuLeuSerCysLeuMetLeuLeuSerGlnValGln

GlyGluAspSerProLysLysIleProSerAlaArgIleSerCysProLysGlySerGlnAlaTyrGlySerTyr

CysTyrAlaLeuPheGlnIleProGlnThrTrpPheAspAlaGluLeuAlaCysGlnLysArgProGluGlyHis

LeuValSerValLeuAsnValAlaGluAlaSerPheLeuAlaSerMetValLysAsnThrGlyAsnSerTyrGln

TyrThrTrpIleGlyLeuHisAspProThrLeuGlyGlyGluProAsnGlyGlyGlyTrpGluTrpSerAsnAsn

AspIleMetAsnTyrValAsnTrpGluArgAsnProSerThrAlaLeuAspArgGlyPheCysGlySerLeuSer

ArgSerSerGlyPheLeuArgTrpArgAspThrThrCysGluValGluValAlaLeuArgLeuGlnIleTyrArg

LeuLysLeuProAspSerLysGlnLeu

Séquence A2 :

GluAspSerProLysLysIleProSerAlaArgIleSerCysProLysGlySerGlnAlaTyrGlySerTyr

CysTyrAlaLeuPheGlnIleProGlnThrTrpPheAspAlaGluLeuAlaCysGlnLysArgProGluGlyHis

LeuValSerValLeuAsnValAlaGluAlaSerPheLeuAlaSerMetValLysAsnThrGlyAsnSerTyrGln

TyrThrTrpIleGlyLeuHisAspProThrLeuGlyGlyGluProAsnGlyGlyGlyTrpGluTrpSerAsnAsn

AspIleMetAsnTyrValAsnTrpGluArgAsnProSerThrAlaLeuAspArgGlyPheCysGlySerLeuSer

ArgSerSerGlyPheLeuArgTrpArgAspThrThrCysGluValGluValAlaLeuArgLeuGlnIleTyrArg

LeuLysLeuProAspSerLysGlnLeu

Une seconde famille de fragments d'ADN selon l'invention comprend des fragments d'ADNc d'une protéine PAP humaine, tels qu'obtenus par la mise en oeuvre des étapes suivantes :
- un premier criblage d'une bibliothèque d'ADNc pancréatique humaine, ledit ADNc humain étant inséré dans un vecteur de clonage approprié, comprenant l'hybridation avec des sondes constituées par l'ADNc de la protéine PAP du rat tel que représenté par les séquences S1 ou S2, dans une solution constituée par : 6 x SSC, 5 x Denhart, 0,5 % SDS, 10 mM EDTA, 200 µg ADN de sperme de saumon, pendant 18 heures à 68°C suivie d'un rinçage dans les conditions suivantes 6 x SSC, 0,1 % SDS, 2 fois 15 minutes à 65°C,
- la sélection des clones positifs d'ADNc humain ayant hybridé lors du criblage avec l'ADNc de la protéine PAP de rat, ces clones étant dits positifs,
- un deuxième criblage avec une séquence d'ADNc d'une protéine PSP dans les conditions d'hybridation ci-dessus avec un rinçage en présence de 0,1 x SSC, 0,1 % SDS, pendant 2 heures à 65°C, afin d'éliminer les clones non spécifiques de l'ADNc de PAP humaine ayant cependant hybridé avec l'ADNc de PAP de rat et
- la récupération des clones n'ayant pas hybridé avec l'ADNc PSP,
- la récupération des fragments d'ADNc des clones positifs obtenus.

Un vecteur de clonage particulièrement avantageux pouvant être modifié par l'ADNc humain pour la réalisation de la bibliothèque d'ADNc pancréatique humaine, est le vecteur λgt10.

La PSP ou "Pancreatic Stone Protein" est une protéine qui présente certaines analogies structurales avec la PAP de rat. Cette protéine a été décrite par Giorgi et al, J. Clin. Invest. (1989), **84**, 100-106.

Les fragments d'ADNc ainsi définis sont caractéristiques de la famille de protéines comprenant la protéine PAP humaine.

Selon un mode de réalisation particulier de l'invention, des fragments préférés d'ADNc sont tels qu'obtenus par la mise en oeuvre des étapes précédentes auxquelles est ajoutée l'étape suivante, avant la récupération des fragments d'ADNc des clones positifs obtenus : criblage avec l'ADNc de PAP de rat dans des conditions d'hybridation telles que celles décrites ci-dessus, avec un rinçage pendant 2 heures à 65°C.

Selon un mode de réalisation avantageux de l'invention, un fragment d'ADNc codant pour la PAP humaine répond à l'enchaînement S3 suivant :

ATG CTG
CCT CCC ATG GCC CTG CCC AGT GTA TCT TGG ATG CTG CTT
TCC TGC CTC ATG CTG CTG TCT CAG GTT CAA GGT GAA GAA
CCC CAG AGG GAA CTG CCC TCT GCA CGG ATC CGC TGT CCC
AAA GGC TCC AAG GCC TAT GGC TCC CAC TGC TAT GCC TTG
TTT TTG TCA CCA AAA TCC TGG ACA GAT GCA GAT CTG GCC
TGC CAG AAG CGG CCC TCT GGA AAC CTG GTG TCT GTG CTC
AGT GGG GCT GAG GGA TCC TTC GTG TCC TCC CTG GTG AAG
AGC ATT GGT AAC AGC TAC TCA TAC GTC TGG ATT GGG CTC
CAT GAC CCC ACA CAG GGC ACC GAG CCC AAT GGA GAA GGT
TGG GAG TGG AGT AGC AGT GAT GTG ATG AAT TAC TTT GCA
TGG GAG AGA AAT CCC TCC ACC ATC TCA AGC CCC GGC CAC
TGT GCG AGC CTG TCG AGA AGC ACA GCA TTT CTG AGG TGG
AAA GAT TAT AAC TGT AAT GTG AGG TTA CCC TAT GTC TGC
AAA GTT CAC

L'invention vise aussi le fragment d'ADNc S4 qui comprend la séquence S3 ainsi que les enchaînements d'ADN suivants respectivement à ses extrémités NH₂ - et COOH - terminales : cgggagagtgactcctgattgcctcctcaagtcgcagacact et,

tgactagtgcaggagggaagtcagcagcctgtgtttggt
gtgcaactcatcatgggcatgagaccagtgtgaggactcaccctggaagaga
atattcgcttaattcccccaacctgaccacctcattcttatctttcttctgt
ttcttcctccccgctagtcatttcagtctcttcattttgtcatacggcctaa
ggctttaaagagcaataaattttagtctgcaaaaaa

Selon un autre mode de réalisation de l'invention, l'ADNc de la PAP humaine est caractérisé en ce qu'il code pour la protéine répondant à la séquence d'acides aminés A3 suivante :

Selon une autre définition, des fragments d'ADNc de la deuxième famille, un fragment d'ADNc selon l'invention est caractérisé en ce qu'il comprend une séquence nucléotidique présentant une homologie d'au moins 60 %, de préférence d'au moins 70 % avec au moins un enchaînement d'environ 100 nucléotides, compris dans la séquence S2 suivante caractéristique de l'ADNc de la PAP mature de rat, ou dans la séquence S3 donnée ci-dessus et caractéristique d'un fragment d'ADNc de PAP humaine.

Séquence S2 :

10 20 30 40 50 60
GAAGACT CTCCGAAGAA AATACCCTCT GCACGCATTA GTTGCCCCAA AGGCTCCCAG
70 80 90 100 110 120
GCATATGGCT CCTACTGCTA TGCCCTGTTT CAGATACCAC AGACCTGGTT TGATGCAGAA
130 140 150 160 170 180
CTGGCCTGCC AGAAGAGACC TGAAGGACAC CTTGTATCTG TGCTCAATGT AGCTGAAGCT
190 200 210 220 230 240
TCATTCTTGG CATCCATGGT CAAGAACACT GGAAACAGCT ACCAATATAC CTGGATTGGA
250 260 270 280 290 300
CTCCATGACC CCACTCTTGG TGGAGAACCC AATGGAGGTG GATGGGAGTG GAGTAACAAT
310 320 330 340 350 360
GACATAATGA ATTATGTCAA CTGGGAGAGG AACCCATCTA CTGCCCTAGA CCGCGGATTC
370 380 390 400 410 420
TGTGGCAGCT TGTCAAGATC TTCTGGATTT CTAAGATGGA GAGATACCAC ATGTGAAGTT
430 440 450 460 470 480
GAAGTTGCCC TACGTCTGCA AATTTACAGG TTAAAATTAC CAGACAGCAA ACAGCTT

L'invention vise aussi des fragments d'ADNc codant pour la PAP humaine caractérisés par leur capacité d'hybridation avec la séquence nucléotidique S1 caractéristique de l'ADNc de la PAP de rat, et/ou avec la séquence nucléotidique S2 caractéristique de l'ADNc de la PAP mature de rat, dans une solution d'hybridation contenant 6 x SSC, 5 x Denhart, 0,5 % SDS, 10 mM EDTA, 200 µg ADN de sperme de saumon, pendant 18 heures à 68°C et avec un rinçage dans une solution comprenant 6 x SSC, 0,1 % SDS, 2 fois pendant 15 minutes à 65°c.

Un fragment d'ADNc de la PAP humaine particulièrement préféré dans le cadre de la présente demande, est caractérisé en ce qu'il comprend la séquence nucléotidique suivante:

                                                10                    20                    30                    40                    50                    60
TTTGTTAAGG ATTCCCTTGA GAATTATGTA AAAGTTTTAC AAGAGTCCAT CTCATTCTCT

                                                70                    80                    90                   100                  110                  120
TTGTCCCCCT CAAAGCTGGC TTGCCAGAAG CGGCCCTCTG GAAAACTGGT GTCTGTGCTC

                                               130                  140                  150                  160                  170                  180
AGTGGGGCTG AGGGATCCTT CGTGTCCTCC CTGGTGAGGA GCATTAGTAA CAGCTACTCA

                                               190                  200                  210                  220                  230                  240
TACATCTGGA TTGGGCTCCA TGACCCCACA CAGGTGCGAG TATATCCTCC CCTCTCTGTT

                                               250                  260                  270                  280                  290                  300
ACCTCTCAAG GTACTGTTGT TGCCCAGGCG CACTCCCTGT CCCCAGTCCC TGCCCAGGAA

GTACTT

Les inventeurs ont constaté que la séquence protéique déduite de cette séquence nucléotidique humaine présente certaines homologies avec la séquence codant pour la protéine PAP de rat, bien que la seule connaissance jusqu'à présent des anticorps dirigés contre la PAP de rat ne permettait pas de détecter la protéine PAP humaine dans un échantillon biologique donné.

L'identification d'un fragment d'ADNc codant pour la protéine PAP humaine permet maintenant d'envisager la production de cette protéine, notamment par voie de génie génétique, ainsi que l'obtention d'anticorps, utilisables en tant que moyens de diagnostic d'une pancréatique aiguë.

L'invention concerne également un fragment d'acide nucléique caractérisé en ce qu'il s'agit du fragment d'ADN complémentaire des fragments d'ADNc ci-dessus définis, ou encore en ce qu'il s'agit du fragment d'ARN correspondant à ces ADNc.

L'invention vise aussi tout fragment de séquence nucléotidique codant pour la PAP humaine, susceptible d'être utilisé comme sonde, après un marquage approprié dudit fragment, en vue de réaliser la détection l'acide nucléique caractéristique de la PAP humaine dans un échantillon biologique.

L'invention concerne également la protéine PAP humaine telle qu'obtenue par l'expression d'un fragment d'ADNc de la PAP humaine, tel que défini ci-dessus, au moyen d'un système d'expression adapté, par exemple un hôte cellulaire transformé par un vecteur d'expression, lui-même modifié par l'insertion du susdit fragment d'ADNc de la PAP humaine.

Une protéine PAP humaine particulière selon l'invention est une protéine telle qu'obtenue par l'expression d'un fragment d'ADNc de la PAP humaine, inséré en phase dans un vecteur pEX, dans E.coli, notamment dans E.coli souche pop2136.

Des protéines PAP humaines selon l'invention sont aussi caractérisées en ce que leurs séquences d'acides aminés présentent une homologie d'au moins 50%, de préférence d'au moins 60% et de façon très préférée d'au moins 70 % avec au moins un enchaînement d'environ 25 acides aminés, compris dans la séquence A2 de la protéine PAP de rat mature, ou dans la séquence A3 de la protéine PAP humaine ci-dessus décrite.

Selon un mode de réalisation particulier de l'invention, la protéine PAP humaine répond à la séquence A3 donnée dans les pages précédentes.

L'invention vise aussi tout fragment de la séquence A3 dès lors qu'il est reconnu par des anticorps reconnaissant cette séquence, notamment des anticorps monoclonaux dirigés spécifiquement contre la séquence A3.

La protéine PAP humaine peut être produite par les techniques du génie génétique ou encore par purification, par exemple par chromatographie, à partir d'un échantillon biologique la contenant .

Selon une définition particulière de l'invention, la protéine PAP humaine répondant aux définitions précédentes, comprend la séquence d'acides aminés suivante :

39
Phe Val Lys Asp Ser Leu Glu Asn Tyr Val Lys Val Leu 13

78
Gln Glu Ser Ile Ser Phe Ser Leu Ser Pro Ser Lys Leu 26

117
Ala Cys Gln Lys Arg Pro Ser Gly Lys Leu Val Ser Val 39

156
Leu Ser Gly Ala Glu Gly Ser Phe Val Ser Ser Leu Val 52

195
Arg Ser Ile Ser Asn Ser Tyr Ser Tyr Ile Trp Ile Gly 65

234
Leu His Asp Pro Thr Gln Val Arg Val Tyr Pro Pro Leu 78

273
Ser Val Thr Ser Gln Gly Thr Val Val Ala Gln Ala His 91

306
Ser Leu Ser Pro Val Pro Ala Gln Glu Val Leu 102

Entre également dans le champ de la présente invention, la protéine PAP de rat caractérisée en ce qu'elle répond à l'enchaînement d'acides aminés A2 ci-dessus, ou une variante ou une partie de cette séquence dès lors que cette partie ou cette variante est reconnue par des anticorps dirigés contre la protéine PAP de rat ou qu'elle présente une homologie d'au moins 50 %, de préférence d'au moins 60 % avec la séquence A2 de PAP de rat ci-dessus, ou avec la séquence A3 de la PAP humaine.

L'invention vise aussi la protéine PAP de rat, répondant à la séquence A1 ou à une variante de A1 présentant les caractéristiques définies ci-dessus vis-à-vis de la séquence d'acides aminés A2.

Chez le rat la PAP est présente en très faible quantité au niveau d'un pancréas humain normal, et sa synthèse peut être considérablement augmentée en cas de pancréatite aiguë jusqu'à un niveau d'environ 50 à 100 fois supérieur à celui qui existe dans le pancréas normal.

La protéine PAP de rat subit une augmentation de son taux dans le suc pancréatique lors d'une pancréatite aiguë, alors que le taux des autres enzymes diminue.

De plus, dans la pancréatite, toutes les protéines secrétoires pancréatiques passent dans le sang. Par conséquent, la mise en oeuvre dans le sang ou dans un autre échantillon biologique d'un patient humain (par exemple l'urine ou le liquide péritonéal), du dosage de la protéine PAP dont la synthèse est considérablement augmentée en cas de pancréatite, laisse supposer que l'on aura un différentiel normal/pathologique, beaucoup plus important que lors des tests habituellement utilisés pour la détection, et donc beaucoup plus facilement décelable.

L'invention se rapporte aussi dans ce but à des anticorps caractérisés en ce qu'ils reconnaissent la protéine PAP humaine précédemment définie, ces anticorps pouvant être soit polyclonaux soit monoclonaux.

Des anticorps monoclonaux sont par exemple des anticorps tels que produits par un hybridome préalablement formé par fusion d'une cellule de myélome avec une cellule splénique d'un animal préalablement immunisé avec une protéine PAP humaine.

L'invention concerne aussi les hybridomes formés par fusion de cellules de myélome et de cellules spléniques d'animal préalablement immunisé avec la protéine PAP humaine, notamment avec la protéine correspondant à l'enchaînement d'acides aminés A3.

Des anticorps monoclonaux particulièrement avantageux dans le cadre de l'invention, sont ceux qui reconnaissent de façon spécifique la partie NH₂-terminale de la PAP humaine. Des anticorps particuliers sont également définis en ce qu'ils reconnaissent la PAP humaine et ce qu'ils sont dépourvus de réaction immunologique avec les autres lectines.

A titre d'exemple, un anticorps monoclonal intéressant notamment pour la détection de la PAP humaine est un anticorps monoclonal dirigé contre le peptide suivant de la PAP humaine : Glu Glu Pro Gln Arg. Pour suivre ce peptide dans des tests de détection de la PAP humaine on a par exemple ajouté un résidu tyrosine sur l'arginine de façon à rendre possible le marquage à l'Iode selon les techniques habituelles de marquage.

L'invention concerne aussi des anticorps antiidiotypiques dirigés contre les déterminants antigéniques des anticorps de l'invention reconnaissant la PAP humaine.

D'autres anticorps selon l'invention sont des anticorps monoclonaux qui reconnaissent la protéine PAP de rat.

Un protocole d'immunisation d'animaux choisis, notamment de souris ou de lapins, pour la mise en oeuvre de l'invention, est le protocole décrit par Kohler et Milstein, Nature (1975), 256, 495-497.

Entre également dans le cadre de l'invention, un vecteur d'expression et/ou de clonage, caractérisé en ce qu'il comprend un fragment d'ADN choisi parmi les fragments précédemment définis.

Des vecteurs d'expression et/ou de clonage particulièrement avantageux pour la mise en oeuvre de l'invention comprennent le plasmide d'expression pEX capable d'exprimer l'ADNc de la protéine PAP humaine dans une bactérie, par exemple E.coli.

D'autres vecteurs sont choisis en fonction de l'hôte chez lequel on recherche l'expression. A cet égard, il peut s'agir pour les cellules de mammifères, d'un vecteur de type baculovirus.

L'invention se rapporte également à un hôte cellulaire transformé par un vecteur d'expression tel que précédemment défini, dans des conditions permettant l'obtention de la protéine ou du peptide codé par le fragment d'ADN de l'invention, inséré dans ce vecteur.

A titre d'exemple, des hôtes cellulaires formant un système d'expression adéquat pour les fragments d'ADN de l'invention, sont des bactéries telles que E.coli, notamment la souche pop2136.

L'invention concerne également le produit d'expression de l'hôte cellulaire transformé selon ce qui précède.

Avantageusement on choisira d'exprimer le fragment d'ADN selon l'invention dans un hôte procaryote ou eucaryote en fonction du produit que l'on souhaite avoir s'agissant notamment de sa glycosylation.

A titre d'exemple, des hôtes cellulaires intéressants pour la mise en oeuvre de l'invention, sont des bactéries, par exemple E.coli, des levures, des cellules d'insectes ou de mammifères, par exemple des cellules CHO.

La présente invention concerne également des compositions, caractérisées en ce qu'elles comprennent au moins un anticorps choisi parmi ceux qui ont été précédemment définis, dirigés contre la protéine PAP humaine. Une telle composition peut être une composition pour le diagnostic in vitro, pour la mise en oeuvre sur un échantillon biologique tel que le sang, l'urine ou le liquide péritonéal d'un patient manifestant des symptômes de la pancréatite aiguë.

Les anticorps utilisés seront le cas échéant marqués par des marqueurs chimiques adaptés.

L'invention vise aussi un kit pour le diagnostic in vitro de la pancréatite aiguë à partir d'un échantillon biologique déterminé, caractérisé en ce qu'il comprend:
- au moins un anticorps choisi parmi les précédents capable de détecter la présence d'un antigène de type PAP humaine dans ledit échantillon biologique, lesdits anticorps étant marqués,
- selon la nature du marquage, un réactif pour détecter la présence d'un complexe du type antigène-anticorps spécifique,
- un témoin négatif.

De préférence les anticorps mis en oeuvre sont spécifiques de la PAP humaine dès lors qu'ils sont dépourvus de réaction avec les autres lectines connues.

Pour effectuer le marquage des anticorps selon l'invention, on pourra avoir recours par exemple à des radioisotopes, à des marqueurs chimiques ou enzymatiques, à des marqueurs chimioluminescents.

L'invention se rapporte par ailleurs à un procédé pour détecter in vitro une pancréatite aiguë à partir d'un échantillon biologique comprenant les étapes de:
- mise en contact de l'échantillon biologique susceptible de contenir la PAP humaine avec des anticorps dirigés contre la PAP humaine,
- détection de la réaction antigène-anticorps entre les susdits anticorps et la PAP humaine.

L'invention vise aussi l'utilisation des anticorps dirigés contre la PAP le cas échéant sous forme de composition comprenant plusieurs anticorps marqués, pour la visualisation en imagerie médicale de la glande pancréatique, l'anticorps étant au préalable marqué à l'aide d'un radioisotope ou d'un marqueur chimique ou enzymatique.

La visualisation peut permettre de déceler la présence de PAP humaine.

A cet égard, l'invention vise un procédé d'observation de la glande pancréatique caractérisé par :
- l'injection à un patient d'une composition ci-dessus, dans des conditions physiologiquement acceptables,
- l'observation d'une réaction entre les anticorps contenus dans la susdite composition et la PAP humaine lorsqu'elle est présente.

D'autres caractéristiques et avantages de l'invention apparaîtront dans les exemples et les figures qui suivent :

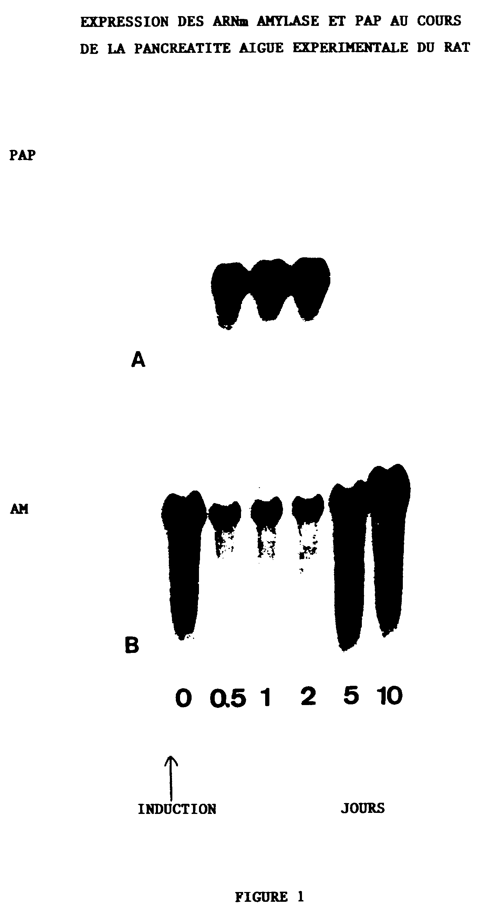

- figure 1 : Expression de la PAP et de l'amylase au cours de la pancréatite aiguë expérimentale du rat. Analyse par "Northern blot" - charge par piste : 30 µg ARN total, le même filtre a été utilisé successivement pour PAP et Amylase. Sondes : cDNA PAP (environ 800 bp) et Amylase (environ 1100 bp), marquées au ³P avec une activité spécifique d'environ 2x10⁹ cpm/mg.
- on a observé les phénomènes suivants : induction de l'expression du gène pap (12 heures -48 heures) (phase aiguë) ; suppression de l'induction durant la récupération (5-10 jours) ; au contraire, l'amylase baisse durant la phase aiguë.

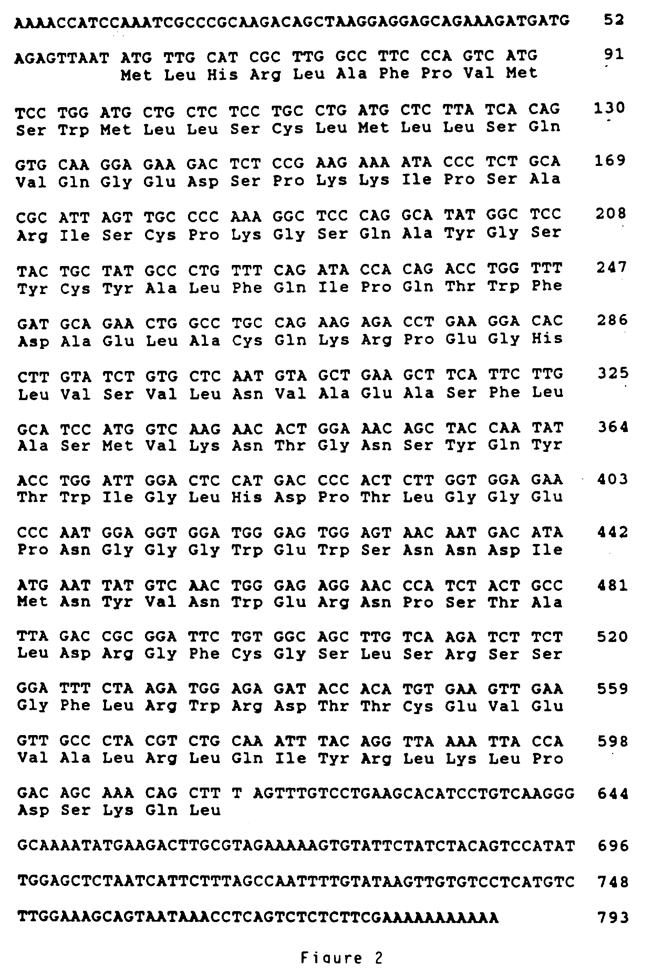

- figure 2 : Séquence nucléotidique codant pour la PAP de rat, et la séquence d'acides aminés correspondante.

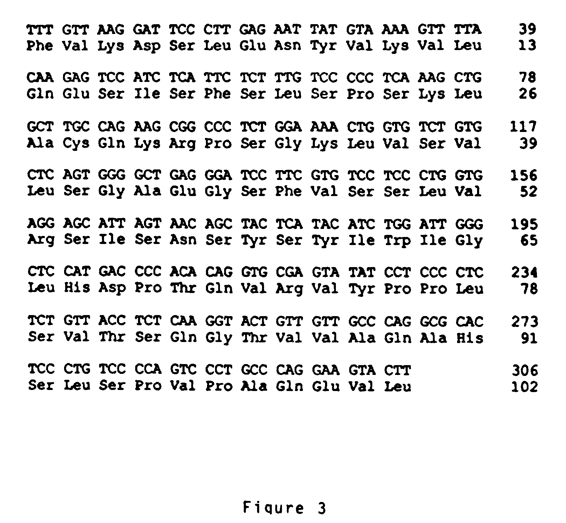

- figure 3 : Fragment de la séquence nucléotidique codant pour la PAP humaine, et la séquence d'acides aminés correspondante.

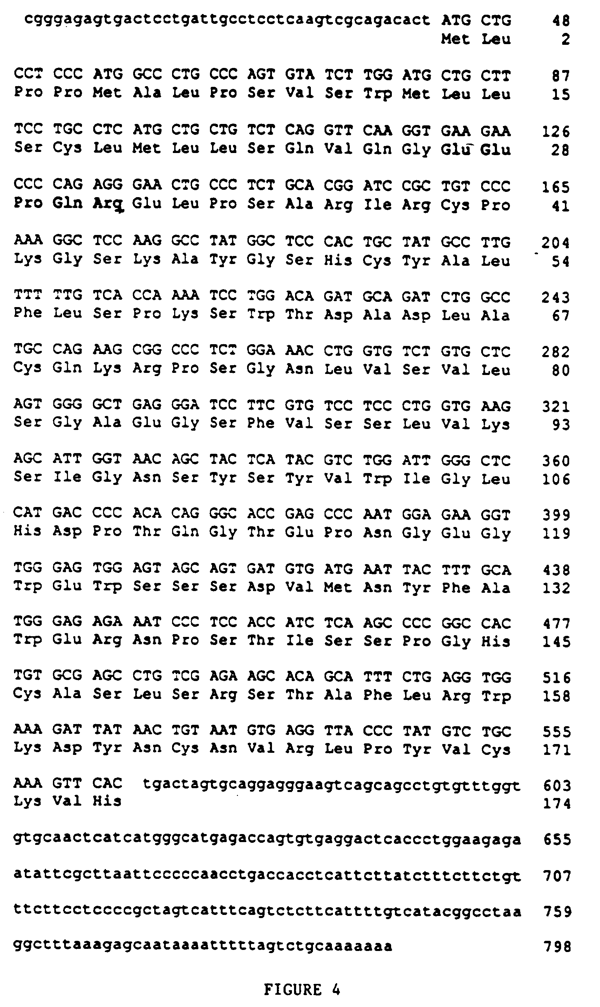

- figure 4: Séquence nucléotidique (S4) caractéristique de la PAP humaine dont la séquence codante S4, et la séquence d'acides aminés (A3) qui lui correspond.

### 1. construction d'une bibliothèque DNAc pancréatique de rat dans le vecteur λ gt11.

Préparation de l'ARN pancréatique de rat : la préparation a été réalisée en suivant exactement la technique de Chirgwin, J.M., et al, Biochemistry (WASH) (1979), 19, 5294-5299. La fraction des ARN messagers de cet ARN total a été séparée par chromatographie sur colonne d'oligo-dT cellulose selon la technique de Aviv, H. et Leder, P., Proc. Natl. Acad. Sci., USA, (1972), 69, 1408-1412.

Préparation du cDNA : l'ADNc prancréatique a été synthétisé à l'aide du kit commercialisé par Amersham France S.A. (code RPN 1256), en suivant exactement les recommandations du fabricant.

Construction de la bibliothèque : la bibliothèque a été construite dans le vecteur d'expression λgt11 à l'aide du kit commercialisé par Amersham France S.A. (code RPN 1280), en suivant exactement les recommandations du fabricant.

### 2. Construction d'une bibliothéque d'ADNc pancréatique humaine dans le vecteur λgt10.

Préparation d'ARN pancréatique humain et synthèse de l'ADNc correspondant aux ARN messagers : des fragments de pancréas normal provenant de donneurs d'organe en coma dépassé ont été traités comme décrit ci-dessus pour le pancréas de rat.

Construction de la bibliothèque : la bibliothèque a été construite dans le vecteur de clonage λgt10 à l'aide du kit commercialisé par Amersham France S.A. (code RPN 1257), en suivant les recommandations du fabricant.

### 3. Criblage de la bibliothèque d'ADNc pancréatique humaine avec un clone exprimant la PAP de rat.

A partir de la bibliothèque d'ADNc pancréatique de rat construite selon la méthode ci-dessus, on a sélectionné un clone reconnu par des anticorps polyclonaux dirigés contre la PAP de rat.

L'ADNc de ce clone de PAP de rat a été isolé et utilisé en tant que sonde pour cribler la banque d'ADNc pancréatique humaine obtenue dans λgt10, telle que décrite ci-dessus.

Dans un premier temps, le criblage a été réalisé dans les conditions de stringence suivantes 6 x SSC, 5 x Denhart, 0,5 % SDS, 10 mM EDTA, 200 µg ADN de sperme de saumon, 18 heures à 68°C avec un rinçage dans 6 x SSC, 0,1 % SDS, 2 fois pendant 15 minutes à 65°C. Ceci permet d'obtenir environ 80 clones d'ADNc humain.

Ensuite on a réalisé un criblage avec l'ADNc PSP, dans le but d'éliminer les clones non caractéristiques de la PAP humaine, qui étaient cependant capable d'hybrider avec l'ADNc de la PAP de rat dans les conditions ci-dessus: on a récupéré environ 50 clones qui comportent donc un fragment d'ADNc codant pour une protéine ou un polypeptide appartenant à la famille de la protéine PAP humaine.

Parmi ces clones on a sélectionné ceux dont l'ADNc présente la plus forte homologie avec la protéine PAP de rat, en réalisant un second criblage au moyen de l'ADNc de PAP de rat dans des conditions de stringence suivantes 6 x SSC, 5 x Denhart, 0,5 % SDS, 10 mM EDTA, 200 µg ADN de sperme de saumon, 18 heures à 68° avec un rinçage dans 6 x SSC, 0,1 % SDS, pendant 2 heures à 65°C. On a ainsi sélectionné 9 clones positifs.

### 4. Séquençage des clones sélectionnés

Les insertions des clones intéressants ont été sous-clonées dans les phages M13mp18-M13mp19 comme décrit dans "Molecular Cloning, a laboratory manual" Sambrook , J., Fritsch, E.F., et Maniatis T. eds, Cold Spring Harbor Laboratory Press (1990). Le séquençage des phages M13 recombinants a été réalisé par la technique décrite par Sanger F. et al, Proc. Natl. Acad. Sci. (USA) (1977), 74, 5463-5467, en utilisant l'amorce universelle commercialisée par Amersham (code N4511).

### 5. Expression d'un fragment de PAP dans E.coli à l'aide du plasmide d'expression pEX et préparation d'anticorps contre la protéine hybride.

Un kit d'expression faisant appel au plasmide pEX dans E. coli, commercialisé par la société Genofit sous la référence G2104, a été utilisé en suivant les recommandations du fabricant. Des fragments de restriction correspondant à la séquence codante de la protéine ont été insérés dans le plasmide pEX. Les plasmides recombinants ont servi à transformer des bactéries (E. coli souche pop 2136). Les protéines des bactéries recombinantes ont été analysées par électrophorèse en gel de polyacrylamide à 7,5 %. La bande protéique correspondant à la PAP hybride a été découpée, homogénéisée dans de l'adjuvant de Freund et injectée à des lapins à raison de 3 injections par lapin à 3 semaines d'intervalle, chaque injection contenant environ 20 µg de protéine hybride. Le sang des lapins a ensuite été prélevé et les immunoglobulines G purifiées sur colonne de protéine A-Sepharose (Pharmacia-France).

## Revendications

1. Fragment d'ADNc caractéristique d'une protéine PAP humaine, tel qu'obtenu par la mise en oeuvre des étapes suivantes :
- un premier criblage d'une bibliothèque d'ADNc pancréatique humaine, ledit ADNc humain étant inséré dans un vecteur de clonage approprié, comprenant l'hybridation avec des sondes constituées par l'ADNc de la protéine PAP du rat, tel que représenté par les séquences S1 ou S2 suivantes 
Séquence S 1 :

10 20 30 40 50 60
AAAACCATCC AAATCGCCCG CAAGACAGCT AAGGAGGAGC AGAAAGATGA TGAGAGTTAA
70 80 90 100 110 120
TATGTTGCAT CGCTTGGCCT TCCCAGTCAT GTCCTGGATG CTGCTCTCCT GCCTGATGCT
130 140 150 160 170 180
CTTATCACAG GTGCAAGGAG AAGACTCTCC GAAGAAAATA CCCTCTGCAC GCATTAGTTG
190 200 210 220 230 240
CCCCAAAGGC TCCCAGGCAT ATGGCTCCTA CTGCTATGCC CTGTTTCAGA TACCACAGAC
250 260 270 280 290 300
CTGGTTTGAT GCAGAACTGG CCTGCCAGAA GAGACCTGAA GGACACCTTG TATCTGTGCT
310 320 330 340 350 360
CAATGTAGCT GAAGCTTCAT TCTTGGCATC CATGGTCAAG AACACTGGAA ACAGCTACCA
370 380 390 400 410 420
ATATACCTGG ATTGGACTCC ATGACCCCAC TCTTGGTGGA GAACCCAATG GAGGTGGATG
430 440 450 460 470 480
GGAGTGGAGT AACAATGACA TAATGAATTA TGTCAACTGG GAGAGGAACC CATCTACTGC
490 500 510 520 530 540
CTTAGACCGC GGATTCTGTG GCAGCTTGTC AAGATCTTCT GGATTTCTAA GATGGAGAGA
550 560 570 580 590 600
TACCACATGT GAAGTTGAAG TTGCCCTACG TCTGCAAATT TACAGGTTAA AATTACCAGA
610 620 630 640 650 660
CAGCAAACAG CTTTAGTTTG TCCTGAAGCA CATCCTGTCA AGGGGCAAAA TATGAAGACT
670 680 690 700 710 720
TGCGTAGAAA AAGTGTATTC TATCTACAGT CCATATTGGA GCTCTAATCA TTCTTTAGCC
730 740 750 760 770 780
AATTTTGTAT AAGTTGTGTC CTCATGTCTT GGAAAGCAGT AATAAACCTC AGTCTCTCTT
790 800 810 820 830 840
CGAAAAAAAA AAA
 
Séquence S2 :

10 20 30 40 50 60
GAAGACT CTCCGAAGAA AATACCCTCT GCACGCATTA GTTGCCCCAA AGGCTCCCAG
70 80 90 100 110 120
GCATATGGCT CCTACTGCTA TGCCCTGTTT CAGATACCAC AGACCTGGTT TGATGCAGAA
130 140 150 160 170 180
CTGGCCTGCC AGAAGAGACC TGAAGGACAC CTTGTATCTG TGCTCAATGT AGCTGAAGCT
190 200 210 220 230 240
TCATTCTTGG CATCCATGGT CAAGAACACT GGAAACAGCT ACCAATATAC CTGGATTGGA
250 260 270 280 290 300
CTCCATGACC CCACTCTTGG TGGAGAACCC AATGGAGGTG GATGGGAGTG GAGTAACAAT
310 320 330 340 350 360
GACATAATGA ATTATGTCAA CTGGGAGAGG AACCCATCTA CTGCCCTTAGA CCGCGGATTC
370 380 390 400 410 420
TGTGGCAGCT TGTCAAGATC TTCTGGATTT CTAAGATGGA GAGATACCAC ATGTGAAGTT
430 440 450 460 470 480
GAAGTTGCCC TACGTCTGCA AATTTACAGG TTAAAATTAC CAGACAGCAA ACAGCTT
 dans une solution constituée par : 6 x SSC, 5 x Denhart, 0,5 % SDS, 10 mM EDTA, 200 µg ADN de sperme de saumon, pendant 18 heures à 68°C, suivie d'un rinçage dans les conditions suivantes : 6 x SSC, 0,1 % SDS, 2 fois 15 minutes à 65°C,
- la sélection des clones positifs d'ADNc humain ayant hybridé lors du criblage avec l'ADNc de la protéine PAP de rat, ces clones étant dits positifs,
- un deuxième criblage avec une séquence d'ADNc d'une protéine PSP ("Pancreatic Stone Protein") dans les conditions d'hybridation ci-dessus avec un rinçage en présence de 0,1 x SSC, 0,1 % SDS, pendant 2 heures à 65°C afin d'éliminer les clones non spécifiques de l'ADNc de PAP humaine ayant cependant hybridé avec l'ADNc de PAP de rat et
- la récupération des clones n'ayant pas hybridé avec l'ADNc PSP,
- la récupération des fragments ADNc des clones positifs obtenus.

2. Fragment d'ADNc caractéristique d'une protéine PAP humaine selon la revendication 1, caractérisé en ce que l'étape de criblage avec l'ADNc PSP ("Pancreatic Stone Protein") est suivie d'une étape de criblage avec l'ADNc de PAP de rat tel que représenté par les séquences S1 et S2 suivantes 
Séquence S 1 :

10 20 30 40 50 60
AAAACCATCC AAATCGCCCG CAAGACAGCT AAGGAGGAGC AGAAAGATGA TGAGAGTTAA
70 80 90 100 110 120
TATGTTGCAT CGCTTGGCCT TCCCAGTCAT GTCCTGGATG CTGCTCTCCT GCCTGATGCT
130 140 150 160 170 180
CTTATCACAG GTGCAAGGAG AAGACTCTCC GAAGAAAATA CCCTCTGCAC GCATTAGTTG
190 200 210 220 230 240
CCCCAAAGGC TCCCAGGCAT ATGGCTCCTA CTGCTATGCC CTGTTTCAGA TACCACAGAC
250 260 270 280 290 300
CTGGTTTGAT GCAGAACTGG CCTGCCAGAA GAGACCTGAA GGACACCTTG TATCTGTGCT
310 320 330 340 350 360
CAATGTAGCT GAAGCTTCAT TCTTGGCATC CATGGTCAAG AACACTGGAA ACAGCTACCA
370 380 390 400 410 420
ATATACCTGG ATTGGACTCC ATGACCCCAC TCTTGGTGGA GAACCCAATG GAGGTGGATG
430 440 450 460 470 480
GGAGTGGAGT AACAATGACA TAATGAATTA TGTCAACTGG GAGAGGAACC CATCTACTGC
490 500 510 520 530 540
CTTAGACCGC GGATTCTGTG GCAGCTTGTC AAGATCTTCT GGATTTCTAA GATGGAGAGA
550 560 570 580 590 600
TACCACATGT GAAGTTGAAG TTGCCCTACG TCTGCAAATT TACAGGTTAA AATTACCAGA
610 620 630 640 650 660
CAGCAAACAG CTTTAGTTTG TCCTGAAGCA CATCCTGTCA AGGGGCAAAA TATGAAGACT
670 680 690 700 710 720
TGCGTAGAAA AAGTGTATTC TATCTACAGT CCATATTGGA GCTCTAATCA TTCTTTAGCC
730 740 750 760 770 780
AATTTTGTAT AAGTTGTGTC CTCATGTCTT GGAAAGCAGT AATAAACCTC AGTCTCTCTT
790 800 810 820 830 840
CGAAAAAAAA AAA
 
Séquence S2 :

10 20 30 40 50 60
GAAGACT CTCCGAAGAA AATACCCTCT GCACGCATTA GTTGCCCCAA AGGCTCCCAG
70 80 90 100 110 120
GCATATGGCT CCTACTGCTA TGCCCTGTTT CAGATACCAC AGACCTGGTT TGATGCAGAA
130 140 150 160 170 180
CTGGCCTGCC AGAAGAGACC TGAAGGACAC CTTGTATCTG TGCTCAATGT AGCTGAAGCT
190 200 210 220 230 240
TCATTCTTGG CATCCATGGT CAAGAACACT GGAAACAGCT ACCAATATAC CTGGATTGGA
250 260 270 280 290 300
CTCCATGACC CCACTCTTGG TGGAGAACCC AATGGAGGTG GATGGGAGTG GAGTAACAAT
310 320 330 340 350 360
GACATAATGA ATTATGTCAA CTGGGAGAGG AACCCATCTA CTGCCTTAGA CCGCGGATTC
370 380 390 400 410 420
TGTGGCAGCT TGTCAAGATC TTCTGGATTT CTAAGATGGA GAGATACCAC ATGTGAAGTT
430 440 450 460 470 480
GAAGTTGCCC TACGTCTGCA AATTTACAGG TTAAAATTAC CAGACAGCAA ACAGCTT
 dans une solution d'hybridation contenant 6 x SSC, 5 x Denhardt, 0,5 % SDS, 10 mM EDTA, 200 µg d'ADN de sperme de saumon, pendant 18 heures à 68°C, suivi d'un rinçage pendant 2 heures à 65°C.

3. Fragment d'ADNc de la PAP humaine selon l'une quelconque des revendications 1 ou 2, caractérisé en ce qu'il répond à la séquence S3 suivante :

ATG CTG
CCT CCC ATG GCC CTG CCC AGT GTA TCT TGG ATG CTG CTT
TCC TGC CTC ATG CTG CTG TCT CAG GTT CAA GGT GAA GAA
CCC CAG AGG GAA CTG CCC TCT GCA CGG ATC CGC TGT CCC
AAA GGC TCC AAG GCC TAT GGC TCC CAC TGC TAT GCC TTG
TTT TTG TCA CCA AAA TCC TGG ACA GAT GCA GAT CTG GCC
TGC CAG AAG CGG CCC TCT GGA AAC CTG GTG TCT GTG CTC
AGT GGG GCT GAG GGA TCC TTC GTG TCC TCC CTG GTG AAG
AGC ATT GGT AAC AGC TAC TCA TAC GTC TGG ATT GGG CTC
CAT GAC CCC ACA CAG GGC ACC GAG CCC AAT GGA GAA GGT
TGG GAG TGG AGT AGC AGT GAT GTG ATG AAT TAC TTT GCA
TGG GAG AGA AAT CCC TCC ACC ATC TCA AGC CCC GGC CAC
TGT GCG AGC CTG TCG AGA AGC ACA GCA TTT CTG AGG TGG
AAA GAT TAT AAC TGT AAT GTG AGG TTA CCC TAT GTC TGC
AAA GTT CAC

4. Fragment d'ADNc de la PAP humaine selon l'une quelconque des revendications 1 ou 2, caractérisé en ce qu'il code pour l'enchaînement d'acides aminés A3 suivant :

5. Fragment d'ADNc caractéristique d'une protéine PAP humaine selon la revendication 1 ou la revendication 2, caractérisé en ce qu'il comprend une séquence nucléotidique présentant une homologie d'au moins 60 %, de préférence d'au moins 70 % avec au moins un enchaînement d'environ 100 nucléotides, compris dans la séquence S2 suivante caractéristique de l'ADNc de la PAP mature de rat, ou dans la séquence S3 caractéristique d'un fragment d'ADNc de PAP humaine.

Séquence S2 :

10 20 30 40 50 60
GAAGACT CTCCGAAGAA AATACCCTCT GCACGCATTA GTTGCCCCAA AGGCTCCCAG
70 80 90 100 110 120
GCATATGGCT CCTACTGCTA TGCCCTGTTT CAGATACCAC AGACCTGGTT TGATGCAGAA
130 140 150 160 170 180
CTGGCCTGCC AGAAGAGACC TGAAGGACAC CTTGTATCTG TGCTCAATGT AGCTGAAGCT
190 200 210 220 230 240
TCATTCTTGG CATCCATGGT CAAGAACACT GGAAACAGCT ACCAATATAC CTGGATTGGA
250 260 270 280 290 300
CTCCATGACC CCACTCTTGG TGGAGAACCC AATGGAGGTG GATGGGAGTG GAGTAACAAT
310 320 330 340 350 360
GACATAATGA ATTATGTCAA CTGGGAGAGG AACCCATCTA CTGCCTTAGA CCGCGGATTC
370 380 390 400 410 420
TGTGGCAGCT TGTCAAGATC TTCTGGATTT CTAAGATGGA GAGATACCAC ATGTGAAGTT
430 440 450 460 470 480
GAAGTTGCCC TACGTCTGCA AATTTACAGG TTAAAATTAC CAGACAGCAA ACAGCTT

6. Fragment d'ADNc caractéristique d'une protéine PAP humaine selon selon l'une quelconque des revendications 1 ou 2, caractérisé en ce qu'il est capable d'hybrider avec la séquence nucléotidique S1 suivante caractéristique de l'ADNc de la PAP de rat, et/ou avec la séquence nucléotidique S2 caractéristique de l'ADNc de la PAP de rat mature dans une solution d'hybridation contenant 6 x SSC, 5 x Denhart, 0,5 % SDS, 10 mM EDTA, 200 µg ADN de sperme de saumon, pendant 18 heures à 68°C et avec un rinçage dans une solution comprenant 6 x SSC, 0,1 % SDS, 2 fois pendant 15 minutes à 65°C.

Séquence S 1 :

        10         20         30         40         50         60
AAAACCATCC AAATCGCCCG CAAGACAGCT AAGGAGGAGC AGAAAGATGA TGAGAGTTAA
        70         80         90        100        110        120
TATGTTGCAT CGCTTGGCCT TCCCAGTCAT GTCCTGGATG CTGCTCTCCT GCCTGATGCT
       130        140        150        160        170        180
CTTATCACAG GTGCAAGGAG AAGACTCTCC GAAGAAAATA CCCTCTGCAC GCATTAGTTG
       190        200        210        220        230        240
CCCCAAAGGC TCCCAGGCAT ATGGCTCCTA CTGCTATGCC CTGTTTCAGA TACCACAGAC
       250        260        270        280        290        300
CTGGTTTGAT GCAGAACTGG CCTGCCAGAA GAGACCTGAA GGACACCTTG TATCTGTGCT
       310        320        330        340        350        360
CAATGTAGCT GAAGCTTCAT TCTTGGCATC CATGGTCAAG AACACTGGAA ACAGCTACCA
       370        380        390        400        410        420
ATATACCTGG ATTGGACTCC ATGACCCCAC TCTTGGTGGA GAACCCAATG GAGGTGGATG
       430        440        450        460        470        480
GGAGTGGAGT AACAATGACA TAATGAATTA TGTCAACTGG GAGAGGAACC CATCTACTGC
       490        500        510        520        530        540
CTTAGACCGC GGATTCTGTG GCAGCTTGTC AAGATCTTCT GGATTTCTAA GATGGAGAGA
       550        560        570        580        590        600
TACCACATGT GAAGTTGAAG TTGCCCTACG TCTGCAAATT TACAGGTTAA AATTACCAGA
       610        620        630        640        650        660
CAGCAAACAG CTTTAGTTTG TCCTGAAGCA CATCCTGTCA AGGGGCAAAA TATGAAGACT
       670        680        690        700        710        720
TGCCGTAGAA AAGTGTATTC TATCTACAGT CCATATTGGA GCTCTAATCA TTCTTTAGCC
       730        740        750        760        770        780
AATTTTGTAT AAGTTGTGTC CTCATGTCTT GGAAAGCAGT AATAAACCTC AGTCTCTCTT
       790        800        810        820        830        840
CGAAAAAAAA AAA

7. Fragment d'ADNc caractéristique d'une protéine PAP humaine selon la revendication 1 ou la revendication 2, caractérisé en ce qu'il comprend la séquence nucléotidique suivante:

10 20 30 40 50 60
TTTGTTAAGG ATTCCCTTGA GAATTATGTA AAAGTTTTAC AAGAGTCCAT CTCATTCTCT
70 80 90 100 110 120
TTGTCCCCCT CAAAGCTGGC TTGCCAGAAG CGGCCCTCTG GAAAACTGGT GTCTGTGCTC
130 140 150 160 170 180
AGTGGGGCTG AGGGATCCTT CGTGTCCTCC CTGGTGAGGA GCATTAGTAA CAGCTACTCA
190 200 210 220 230 240
TACATCTGGA TTGGGCTCCA TGACCCCACA CAGGTGCGAG TATATCCTCC CCTCTCTGTT
250 260 270 280 290 300
ACCTCTCAAG GTACTGTTGT TGCCCAGGCG CACTCCCTGT CCCCAGTCCC TGCCCAGGAA
GTACTT

8. Fragment d'ADNC caractérisé en ce qu'il répond à la séquence nucléotidique S1 codant pour la protéine PAP de rat, à une partie ou à une variante de cette séquence dès lors que cette partie ou variante code pour une protéine ou un peptide reconnu par des anticorps dirigés contre la protéine PAP de rat ou qu'elle hybride avec la séquence S1 dans une solution d'hybridation contenant 6 x SSC, 5 x Denhart, 0,5 % SDS, 10 mM EDTA, 200 µg ADN de sperme de saumon, 18 heures à 68°C et après un rinçage dans les conditions suivantes : 6 x SSC, 0,1 % SDS, 2 fois 15 minutes à 65°C.

9. Fragment d'ADNc caractérisé en ce qu'il code pour une protéine répondant à l'une des séquences d'acides aminés A1 ou A2 suivantes, ou pour une séquence d'acides aminés ayant une homologie de 40 à 80 % de préférence de 50 à 60 % avec au moins un enchaînement d'environ 25 acides aminés des séquences A1 ou A2, ou dans la séquence A3 de la protéine PAP humaine.

A1

MetLeuHisArgLeuAlaPheProValMetSerTrpMetLeuLeuLeuSerCysLeuMetLeuLeuSerGlnValGln

GlyGluAspSerProLysLysIleProSerAlaArgIleSerCysProLysGlySerGlnAlaTyrGlySerTyr

CysTyrAlaLeuPheGlnIleProGlnThrTrpPheAspAlaGluLeuAlaCysGlnLysArgProGluGlyHis

LeuValSerValLeuAsnValAlaGluAlaSerPheLeuAlaSerMetValLysAsnThrGlyAsnSerTyrGln

TyrThrTrpIleGlyLeuHisAspProThrLeuGlyGlyGluProAsnGlyGlyGlyTrpGluTrpSerAsnAsn

AspIleMetAsnTyrValAsnTrpGluArgAsnProSerThrAlaLeuAspArgGlyPheCysGlySerLeuSer

ArgSerSerGlyPheLeuArgTrpArgAspThrThrCysGluValGluValAlaLeuArgLeuGlnIleTyrArg

LeuLysLeuProAspSerLysGlnLeu

A2

GluAspSerProLysLysIleProSerAlaArgIleSerCysProLysGlySerGlnAlaTyrGlySerTyr

CysTyrAlaLeuPheGlnIleProGlnThrTrpPheAspAlaGluLeuAlaCysGlnLysArgProGluGlyHis

LeuValSerValLeuAsnValAlaGluAlaSerPheLeuAlaSerMetValLysAsnThrGlyAsnSerTyrGln

TyrThrTrpIleGlyLeuHisAspProThrLeuGlyGlyGluProAsnGlyGlyGlyTrpGluTrpSerAsnAsn

AspIleMetAsnTyrValAsnTrpGluArgAsnProSerThrAlaLeuAspArgGlyPheCysGlvSerLeuSer

ArgSerSerGlyPheLeuArgTrpArgAspThrThrCysGluValGluValAlaLeuArgLeuGlnIleTyrArg

LeuLysLeuProAspSerLysGlnLeu

10. Fragment d'acide nucléique caractérisé en ce qu'il s'agit d'un fragment d'ADN complémentaire du fragment d'ADNc selon l'une quelconque des revendications 1 à 9 ou encore en ce qu'il s'agit du fragment d'ARN correspondant à cet ADNc.

11. Protéine PAP humaine, telle qu'obtenue par l'expression d'un fragment d'ADNc de la PAP humaine, tel qu'obtenu selon la revendication 1 ou la revendication 2, au moyen d'un système d'expression adapté, par exemple un hôte cellulaire transformé par un vecteur d'expression, lui-même modifié par l'insertion du susdit fragment d'ADNc de la PAP humaine.

12. Protéine PAP humaine selon la revendication 11, telle qu'obtenue par l'expression d'un fragment d'ADNc de la PAP humaine, inséré en phase dans un vecteur pEX, dans E.coli, notamment dans E.coli souche pop2136.

13. Protéine PAP humaine selon l'une quelconque des revendications 1 à 12, caractérisée en ce qu'elle répond à l'enchaînement d'acides aminés A3.

14. Protéine PAP humaine selon la revendication 11 ou la revendication 12, caractérisée en ce qu'elle comprend l'enchaînement d'acides aminés suivant :

39
Phe Val Lys Asp Ser Leu Glu Asn Tyr Val Lys Val Leu 13

78
Gln Glu Ser Ile Ser Phe Ser Leu Ser Pro Ser Lys Leu 26

117
Ala Cys Gln Lys Arg Pro Ser Gly Lys Leu Val Ser Val 39

156
Leu Ser Gly Ala Glu Gly Ser Phe Val Ser Ser Leu Val 52

195
Arg Ser Ile Ser Asn Ser Tyr Ser Tyr Ile Trp Ile Gly 65

234
Leu His Asp Pro Thr Gln Val Arg Val Tyr Pro Pro Leu 78

273
Ser Val Thr Ser Gln Gly Thr Val Val Ala Gln Ala His 91

306
Ser Leu Ser Pro Val Pro Ala Gln Glu Val Leu 102

15. Protéine PAP humaine caractérisée en ce que sa séquence d'acides aminés présente une homologie d'au moins 50%, de préférence d'au moins 60% et de façon très préférée d'au moins 70 % avec au moins un enchaînement d'environ 25 acides aminés, compris dans la séquence A2 de la protéine PAP de rat mature ou avec la séquence A3 de la protéine PAP humaine.

16. Séquence d'acides aminés, caractérisée en ce qu'il s'agit d'un fragment de la protéine répondant à la séquence d'acides aminés A3, ledit fragment étant reconnu par des anticorps reconnaissant la séquence A3.

17. Séquence d'acides aminés selon la revendication 16, caractérisée en ce qu'elle est reconnue par des anticorps monoclonaux dirigés spécifiquement contre la séquence A3.

18. Protéine PAP de rat caractérisée en ce qu'elle répond à l'enchaînement d'acides aminés suivant, ou une variante ou une partie de cet enchaînement dès lors que cette partie ou cette variante est reconnue par des anticorps dirigés contre la protéine PAP de rat ou qu'elle présente une homologie d'au moins 50 %, de préférence d'au moins 60% avec la séquence A2 de PAP de rat.

A1

MetLeuHisArgLeuAlaPheProValMetSerTrpMetLeuLeuLeuSerCysLeuMetLeuLeuSerGlnValGln

GlyGluAspSerProLysLysIleProSerAlaArgIleSerCysProLysGlySerGlnAlaTyrGlySerTyr

CysTyrAlaLeuPheGlnIleProGlnThrTrpPheAspAlaGluLeuAlaCysGlnLysArgProGluGlyHis

LeuValSerValLeuAsnValAlaGluAlaSerPheLeuAlaSerMetValLysAsnThrGlyAsnSerTyrGln

TyrThrTrpIleGlyLeuHisAspProThrLeuGlyGlyGluProAsnGlyGlyGlyTrpGluTrpSerAsnAsn

AspIleMetAsnTyrValAsnTrpGluArgAsnProSerThrAlaLeuAspArgGlyPheCysGlySerLeuSer

ArgSerSerGlyPheLeuArgTrpArgAspThrThrCysGluValGluValAlaLeuArgLeuGlnIleTyrArg

LeuLysLeuProAspSerLysGlnLeu

19. Anticorps caractérisés en ce qu'ils reconnaissent la protéine PAP humaine selon l'une quelconque des revendications 11 à 16 et en ce qu'il s'agit d'anticorps polyclonaux ou monoclonaux.

20. Anticorps monoclonaux tels qu'obtenus à partir d'un hybridome, précédemment formé par fusion d'une cellule de myélome et d'une cellule splénique d'un animal préalablement immunisé avec une protéine selon l'une quelconque des revendications 11 à 16.

21. Anticorps monoclonaux selon l'une quelconque des revendications 19 ou 20, caractérisés en ce qu'ils reconnaissent spécifiquement la partie NH₂ - terminale de l'enchaînement d'acides aminés A3.

22. Anticorps monoclonaux selon l'une quelconque des revendications 19 à 20, caractérisés en ce qu'ils sont dirigés contre le peptide Glu Glu Pro Gln Arg.

23. Hybridome tel qu'obtenu à partir de la fusion d'une cellule de myélome avec une cellule splénique d'un animal préalablement immunisé avec une protéine PAP humaine selon l'une quelconque des revendications 11 à 16.

24. Vecteur d'expression et/ou de clonage caractérisé en ce qu'il comprend un fragment d'ADN selon l'une quelconque des revendications 1 à 10.

25. Hôte cellulaire transformé par un vecteur d'expression selon la revendication 24, dans des conditions permettant l'obtention de la protéine ou du peptide codé par le fragment d'ADN inséré dans le vecteur, ledit hôte cellulaire étant par exemple choisi parmi les bactéries, notamment en ce qu'il s'agit de E.coli, parmi les levures, les cellules d'insectes ou de mammifères.

26. Produit d'expression de l'hôte cellulaire transformé selon la revendication 25.

27. Composition pour le diagnostic in vitro de la pancréatite aiguë ou pour l'observation de la glande pancréatique en imagerie médicale, dans un échantillon biologique tel que le sang, caractérisée en ce qu'elle comprend au moins un anticorps selon l'une quelconque des revendications 19 à 21 le cas échéant marqué, capable de former un complexe du type anticorps-antigène avec la protéine PAP humaine.

28. Kit pour le diagnostic in vitro de la pancréatite aiguë, à partir d'un échantillon biologique caractérisé en ce qu'il comprend:
- au moins un anticorps selon l'une quelconque des revendications 19 à 22 capable de détecter la présence de la PAP humaine dans ledit échantillon biologique, lesdits anticorps étant le cas échéant marqués,
- selon la nature du marquage, un réactif pour détecter la présence d'un complexe du type antigène-anticorps spécifique,
- un témoin négatif.

29. Procédé pour détecter in vitro une pancréatite aiguë, à partir d'un échantillon biologique comprenant les étapes de:
- mise en contact de l'échantillon biologique susceptible de contenir la PAP humaine, avec des anticorps selon l'une quelconque des revendications 19 à 22,
- détection de la réaction antigène-anticorps entre les susdits anticorps et la PAP humaine.

30. Kit selon la revendication 28 ou procédé selon la revendication 29, caractérisé en ce que les anticorps sont des anticorps polyclonaux.

31. Procédé d'observation de la glande pancréatique caractérisé par :
- l'injection à un patient d'une composition selon la revendication 27, dans des conditions physiologiquement acceptables,
- l'observation notamment par les techniques d'imagerie médicale d'une réaction entre les anticoprs contenus dans la susdite composition et la PAP humaine lorsqu'elle est présente.

## Claims

1. DNAc fragment characteristic of a human PAP protein, such as is obtained by performing the following stages:
- a first screening of a human pancreatic DNAc library, said human DNAc being inserted in an appropriate cloning vector, comprising hybridization with probes constituted by the DNAc of the PAP protein of the rat, as represented by the following sequences S1 or s2 
Séquence S 1 :

10 20 30 40 50 60
AAAACCATCC AAATCGCCCG CAAGACAGCT AAGGAGGAGC AGAAAGATGA TGAGAGTTAA
70 80 90 100 110 120
TATGTTGCAT CGCTTGGCCT TCCCAGTCAT GTCCTGGATG CTGCTCTCCT GCCTGATGCT
130 140 150 160 170 180
CTTATCACAG GTGCAAGGAG AAAGACTCCC GAAGAAAATA CCCTCTGCAC GCATTAGTTG
190 200 210 220 230 240
CCCCAAAGGC TCCCAGGCAT ATGGCTCCTA CTGCTATGCC CTGTTTCAGA TACCACAGAC
250 260 270 280 290 300
CTGGTTTGAT GCAGAACTGG CCTGCCAGAA GAGACCTGAA GGACACCTTG TATCTGTGCT
310 320 330 340 350 360
CAATGTAGCT GAAGCTTCAT TCTTGGCATC CATGGTCAAG AACACTGGAA ACAGCTACCA
370 380 390 400 410 420
ATATACCTGG ATTGGACTCC ATGACCCCAC TCTTGGTGGA GAACCCAATG GAGGTGGATG
430 440 450 460 470 480
GGAGTGGAGT AACAATGACA TAATGAATTA TGTCAACTGG GAGAGGAACC CATCTACTGC
490 500 510 520 530 540
CTTAGACCGC GGATTCTGTG GCAGCTTGTC AAGATCTTCT GGATTTCTAA GATGGAGAGA
550 560 570 580 590 600
TACCACATGT GAAGTTGAAG TTGCCCTACG TCTGCAAATT TACAGGTTAA AATTACCAGA
610 620 630 640 650 660
CAGCAAACAG CTTTAGTTTG TCCTGAAGCA CATCCTGTCA AGGGGCAAAA TATGAAGACT
670 680 690 700 710 720
TGCGTAGAAA AAGTGTATTC TATCTACAGT CCATATTGGA GCTCTAATCA TTCTTTAGCC
730 740 750 760 770 780
AATTTTGTAT AAGTTGTGTC CTCATGTCTT GGAAAGCAGT AATAAACCTC AGTCTCTCTT
790 800 810 820 830 840
CGAAAAAAAA AAA
 
Séquence S2 :

10 20 30 40 50 60
GAAGACT CTCCGAAGAA AATACCCTCT GCACGCATTA GTTGCCCCAA AGGCTCCCAG
70 80 90 100 110 120
GCATATGGCT CCTACTGCTA TGCCCTGTTT CAGATACCAC AGACCTGGTT TGATGCAGAA
130 140 150 160 170 180
CTGGCCTGCC AGAAGAGACC TGAAGGACAC CTTGTATCTG TGCTCAATGT AGCTGAAGCT
190 200 210 220 230 240
TCATTCTTGG CATCCATGGT CAAGAACACT GGAAACAGCT ACCAATATAC CTGGATTGGA
250 260 270 280 290 300
CTCCATGACC CCACTCTTGG TGGAGAACCC AATGGAGGTG GATGGGAGTG GAGTAACAAT
310 320 330 340 350 360
GACATAATGA ATTATGTCAA CTGGGAGAGG AACCCATCTA CTGCCCTAGA CCGCGGATTC
370 380 390 400 410 420
TGTGGCAGCT TGTCAAGATC TTCTGGATTT CTAAGATGGA GAGATACCAC ATGTGAAGTT
430 440 450 460 470 480
GAAGTTGCCC TACGTCTGCA AATTTACAGG TTAAAATTAC CAGACAGCAA ACAGCTT
 in a solution constituted by 6 x SSC, 5 x Denhart, 0.5% SDS, 10 mM EDTA and 200 ug of salmon sperm DNA, for 18 hours at 68°C, followed by a rinsing in the following conditions: 6 x SSC, 0.1% SDS, twice 15 minutes at 65°C,
- the selection of the positive clones of human DNAc having been hybridized during the screening with the DNAc of the rat PAP protein, said clones being called positive,
- the second screening with a DNAc sequence of a PSP (pancreatic stone protein) under the above hybridization conditions with rinsing in the presence of 0.1 x SSC, 0.1% SDS, for 2 hours at 65°C, in order to eliminate the clones not specific of human PAP DNAc, but which have hybridized with the rat PAP DNAc and
- the recovery of the clones which have not hybridized with PSP DNAc and the recovery of the DNAc fragments of the positive clones obtained.

2. DNAc fragment characteristic of a human PAP protein according to claim 1, characterized in that this stage of screening with PSP (pancreatic stone protein) DNAc is followed by a screening stage with rat PAP DNAc, as represented by the following sequences S1 and S2 
Séquence S 1 :

10 20 30 40 50 60
AAAACCATCC AAATCGCCCG CAAGACAGCT AAGGAGGAGC AGAAAGATGA TGAGAGTTAA
70 80 90 100 110 120
TATGTTGCAT CGCTTGGCCT TCCCAGTCAT GTCCTGGATG CTGCTCTCCT GCCTGATGCT
130 140 150 160 170 180
CTTATCACAG GTGCAAGGAG AAGACTCTCC GAAGAAAATA CCCTCTGCAC GCATTAGTTG
190 200 210 220 230 240
CCCCAAAGGC TCCCAGGCAT ATGGCTCCTA CTGCTATGCC CTGTTTCAGA TACCACAGAC
250 260 270 280 290 300
CTGGTTTGAT GCAGAACTGG CCTGCCAGAA GAGACCTGAA GGACACCTTG TATCTGTGCT
310 320 330 340 350 360
CAATGTAGCT GAAGCTTCAT TCTTGGCATC CATGGTCAAG AACACTGGAA ACAGCTACCA
370 380 390 400 410 420
ATATACCTGG ATTGGACTCC ATGACCCCAC TCTTGGTGGA GAACCCAATG GAGGTGGATG
430 440 450 460 470 480
GGAGTGGAGT AACAATGACA TAATGAATTA TGTCAACTGG GAGAGGAACC CATCTACTGC
490 500 510 520 530 540
CTTAGACCGC GGATTCTGTG GCAGCTTGTC AAGATCTTCT GGATTTCTAA GATGGAGAGA
550 560 570 580 590 600
TACCACATGT GAAGTTGAAG TTGCCCTACG TCTGCAAATT TACAGGTTAA AATTACCAGA
610 620 630 640 650 660
CAGCCAAACAG CTTTAGTTTG TCCTGAAGCA CATCCTGTCA AGGGGCAAAA TATGAAGACT
670 680 690 700 710 720
TGCCGTAGAA AAGTGTATTC TATCTACAGT CCATATTGGA GCTCTAATCA TTCTTTAGCC
730 740 750 760 770 780
AATTTTGTAT AAGTTGTGTC CTCATGTCTT GGAAAGCAGT AATAAACCTC AGTCTCTCTT
790 800 810 820 830 840
CGAAAAAAAA AAA
 
Séquence S2 :

10 20 30 40 50 60
GAAGACT CTCCGAAGAA AATACCCTCT GCACGCATTA GTTGCCCCAA AGGCTCCCAG
70 80 90 100 110 120
GCATATGGCT CCTACTGCTA TGCCCTGTTT CAGATACCAC AGACCTGGTT TGATGCAGAA
130 140 150 160 170 180
CTGGCCTGCC AGAAGAGACC TGAAGGACAC CTTGTATCTG TGCTCAATGT AGCTGAAGCT
190 200 210 220 230 240
TCATTCTTGG CATCCATGGT CAAGAACACT GGAAACAGCT ACCAATATAC CTGGATTGGA
250 260 270 280 290 300
CTCCATGACC CCACTCTTGG TGGAGAACCC AATGGAGGTG GATGGGAGTG GAGTAACAAT
310 320 330 340 350 360
GACATAATGA ATTATGTCAA CTGGGAGAGG AACCCATCTA CTGCCCTTAGA CCGCGGATTC
370 380 390 400 410 420
TGTGGCAGCT TGTCAAGATC TTCTGGATTT CTAAGATGGA GAGATACCAC ATGTGAAGTT
430 440 450 460 470 480
GAAGTTGCCC TACGTCTGCA AATTTACAGG TTAAAATTAC CAGACAGCAA ACAGCTT
 in a hybridization solution containing 6 x SSC, 5 x Denhardt, 0.5% SDS, 10 mM EDTA and 200 ug of salmon sperm DNA, for 18 hours at 68°C, followed by rinsing for 2 hours at 65°C.

3. DNAc fragment of human PAP according to either of the claims 1 and 2, characterized in that it complies with the following sequence S3:

ATG CTG
CCT CCC ATG GCC CTG CCC AGT GTA TCT TGG ATG CTG CTT
TCC TGC CTC ATG CTG CTG TCT CAG GTT CAA GGT GAA GAA
CCC CAG AGG GAA CTG CCC TCT GCA CGG ATC CGC TGT CCC
AAA GGC TCC AAG GCC TAT GGC TCC CAC TGC TAT GCC TTG
TTT TTG TCA CCA AAA TCC TGG ACA GAT GCA GAT CTG GCC
TGC CAG AAG CGG CCC TCT GGA AAC CTG GTG TCT GTG CTC
AGT GGG GCT GAG GGA TCC TTC GTG TCC TCC CTG GTG AAG
AGC ATT GGT AAC AGC TAC TCA TAC GTC TGG ATT GGG CTC
CAT GAC CCC ACA CAG GGC ACC GAG CCC AAT GGA GAA GGT
TGG GAG TGG AGT AGC AGT GAT GTG ATG AAT TAC TTT GCA
TGG GAG AGA AAT CCC TCC ACC ATC TCA AGC CCC GGC CAC
TGT GCG AGC CTG TCG AGA AGC ACA GCA TTT CTG AGG TGG
AAA GAT TAT AAC TGT AAT GTG AGG TTA CCC TAT GTC TGC
AAA GTT CAC

4. DNAc fragment of human PAP according to either of the claims 1 and 2, characterized in that it codes for the following A3 amino acid sequence:

5. DNAc fragment characteristic of a human PAP protein according to claim 1 or 2, characterized in that it comprises a nucleotide sequence having a homology of at least 60%, preferably at least 70% with at least one sequence of approximately 100 nucleotides, included in the following sequence S2 characteristic of the DNAc of mature rat PAP, or in the sequence S3 characteristic of a DNAc fragment of human PAP:

Séquence S2 :

10 20 30 40 50 60
GAAGACT CTCCGAAGAA AATACCCTCT GCACGCATTA GTTGCCCCAA AGGCTCCCAG
70 80 90 100 110 120
GCATATGGCT CCTACTGCTA TGCCCTGTTT CAGATACCAC AGACCTGGTT TGATGCAGAA
130 140 150 160 170 180
CTGGCCTGCC AGAAGAGACC TGAAGGACAC CTTGTATCTG TGCTCAATGT AGCTGAAGCT
190 200 210 220 230 240
TCATTCTTGG CATCCATGGT CAAGAACACT GGAAACAGCT ACCAATATAC CTGGATTGGA
250 260 270 280 290 300
CTCCATGACC CCACTCTTGG TGGAGAACCC AATGGAGGTG GATGGGAGTG GAGTAACAAT
310 320 330 340 350 360
GACATAATGA ATTATGTCAA CTGGGAGAGG AACCCATCTA CTGCCTTAGA CCGCGGATTC
370 380 390 400 410 420
TGTGGCAGCT TGTCAAGATC TTCTGGATTT CTAAGATGGA GAGATACCAC ATGTGAAGTT
430 440 450 460 470 480
GAAGTTGCCC TACGTCTGCA AATTTACAGG TTAAAATTAC CAGACAGCAA ACAGCTT

6. DNAc fragment characteristic of a human PAP protein according to either of the claims 1 and 2, characterized in that it is able to hybridize with the following nucleotide sequence S1 characteristic of the DNAc of rat PAP and/or with the nucleotide sequence S2 characteristic of the DNAc of mature rat PAP in a hybridization solution containing 6 x SSC, 5 x Denhart, 0.5% SDS, 10 mM EDTA and 200 ug of salmon sperm DNA, for 18 hours at 68°C and with a rinsing in a solution comprising 6 x SSC, 0,1% SDS, twice during 15 minutes at 65°C:

Séquence S 1 :

10 20 30 40 50 60
AAAACCATCC AAATCGCCCG CAAGACAGCT AAGGAGGAGC AGAAAGATGA TGAGAGTTAA
70 80 90 100 110 120
TATGTTGCAT CGCTTGGCCT TCCCAGTCAT GTCCTGGATG CTGCTCTCCT GCCTGATGCT
130 140 150 160 170 180
CTTATCACAG GTGCAAGGAG AAGACTCTCC GAAGAAAATA CCCTCTGCAC GCATTAGTTG
190 200 210 220 230 240
CCCCAAAGGC TCCCAGGCAT ATGGCTCCTA CTGCTATGCC CTGTTTCAGA TACCACACAC
250 260 270 280 290 300
CTGGTTTGAT GCAGAACTGG CCTGCCAGAA GAGACCTGAA GGACACCTTG TATCTGTGCT
310 320 330 340 350 360
CAATGTAGCT GAAGCTTCAT TCTTGGCATC CATGGTCAAG AACACTGGAA ACAGCTACCA
370 380 390 400 410 420
ATATACCTGG ATTGGACTCC ATGACCCCAC TCTTGGTGGA GAACCCAATG GAGGTGGATG
430 440 450 460 470 480
GGAGTGGAGT AACAATGACA TAATGAATTA TGTCAACTGG GAGAGGAACC CATCTACTGC
490 500 510 520 530 540
CTTAGACCGC GGATTCTGTG GCAGCTTGTC AAGATCTTCT GGATTTCTAA GATGGAGAGA
550 560 570 580 590 600
TACCACATGT GAAGTTGAAG TTGCCCTACG TCTGCAAATT TACAGGTTAA AATTACCAGA
610 620 630 640 650 660
CAGCAAACAG CTTTAGTTTG TCCTGAAGCA CATCCTGTCA AGGGGCAAAA TATGAAGACT
670 680 690 700 710 720
TGCCGTAGAA AAGTGTATTC TATCTACAGT CCATATTGGA GCTCTAATCA TTCTTTAGCC
730 740 750 760 770 780
AATTTTGTAT AAGTTGTGTC CTCATGTCTT GGAAAGCAGT AATAAACCTC AGTCTCTCTT
790 800 810 820 830 840
CGAAAAAAAA AAA

7. DNAc fragment characteristic of a human PAP protein according to claim 1 or 2, characterized in that it comprises the following nucleotide sequence:

        10         20         30         40         50         60
TTTGTTAAGG ATTCCCTTGA GAATTATGTA AAAGTTTTAC AAGAGTCCAT CTCATTCTCT
        70         80         90        100        110        120
TTGTCCCCCT CAAAGCTGGC TTGCCAGAAG CGGCCCTCTG GAAAACTGGT GTCTGTGCTC
       130        140        150        160        170        180
AGTGGGGCTG AGGGATCCTT CGTGTCCTCC CTGGTGAGGA GCATTAGTAA CAGCTACTCA
       190        200        210        220        230        240
TACATCTGGA TTGGGCTCCA TGACCCCACA CAGGTGCGAG TATATCCTCC CCTCTCTGTT
       250        260        270        280        290        300
ACCTCTCAAG GTACTGTTGT TGCCCAGGCG CACTCCCTGT CCCCAGTCCC TGCCCAGGAA

GTACTT

8. DNAc fragment, characterized in that it complies with the nucleotide sequence S1 coding for the rat PAP protein, with part or a variant of said sequence immediately said part or variant codes for a protein or a peptide recognized by antibodies directed against the rat PAP protein or it hybridizes with the sequence S1 in a hybridization solution containing 6 x SSC, 5 x Denhart, 0.5% SDS, 10 mM EDTA and 200 ug salmon sperm DNA, 18 hours at 68°C and after a rinsing under the following conditions: 6 x SSC, 0.1% SDS, twice 15 minutes at 65°C.

9. DNAc fragment, characterized in that it codes for a protein complying with one of the following A1 or A2 amino acid sequences, or for an amino acid sequence having a homology of 40 to 80% and preferably 50 to 60% with at least one sequence of approximately 25 amino acids of sequences A1 or A2, or in sequence A3 of human PAP protein:

A1

MetLeuHisArgLeuAlaPheProValMetSerTrpMetLeuLeuLeuSerCysLeuMetLeuLeuSerGlnValGln

GlyGluAspSerProLysLysIleProSerAlaArgIleSerCysProLysGlySerGlnAlaTyrGlySerTyr

CysTyrAlaLeuPheGlnIleProGlnThrTrpPheAspAlaGluLeuAlaCysGlnLysArgProGluGlyHis

LeuValSerValLeuAsnValAlaGluAlaSerPheLeuAlaSerMetValLysAsnThrGlyAsnSerTyrGln

TyrThrTrpIleGlyLeuHisAspProThrLeuGlyGlyGluProAsnGlyGlyGlyTrpGluTrpSerAsnAsn

AspIleMetAsnTyrValAsnTrpGluArgAsnProSerThrAlaLeuAspArgGlyPheCysGlySerLeuSer

ArgSerSerGlyPheLeuArgTrpArgAspThrThrCysGluValGluValAlaLeuArgLeuGlnIleTyrArg

LeuLysLeuProAspSerLysGlnLeu

λ2

GluAspSerProLysLysIleProSerAlaArgIleSerCysProLysGlySerGlnAlaTyrGlySerTyr

CysTyrAlaLeuPheGlnIleProGlnThrTrpPheAspAlaGluLeuAlaCysGlnLysArgProGluGlyHis

LeuValSerValLeuAsnValAlaGluAlaSerPheLeuAlaSerMetValLysAsnThrGlyAsnSerTyrGln

TyrThrTrpIleGlyLeuHisAspProThrLeuGlyGlyGluProAsnGlyGlyGlyTrpGluTrpSerAsnAsn

AspIleMetAsnTyrValAsnTrpGluArgAsnProSerThrAlaLeuAspArgGlyPheCysGlySerLeuSer

ArgSerSerGlyPheLeuArgTrpArgAspThrThrCysGluValGluValAlaLeuArgLeuGlnIleTyrArg

LeuLysLeuProAspSerLysGlnLeu

10. Nucleic acid fragment, characterized in that it is a DNA fragment complimentary to the DNAc fragment according to any one of the claims 1 to 9, or it is the RNA fragment corresponding to said DNAc.

11. Human PAP protein, such as that obtained by the expression of a DNAc fragment of human PAP, such as is obtained according to claim 1 or 2, by means of an adapted expression system, e.g. a cellular host transformed by an expression vector, itself modified by the insertion of the above human PAP DNAc fragment.

12. Human PAP protein according to claim 11, such as is obtained by the expression of a human PAP DNAc fragment, inserted in phase in a pEX vector in E.coli, particularly in E.coli strain pop2136.

13. Human PAP protein according to any one of the claims 1 to 12, characterized in that it complies wit the A3 amino acid sequence:

14. Human PAP protein according to claim 11 or 12, characterized in that it comprises the following amino acid sequence:

39
Phe Val Lys Asp Ser Leu Glu Asn Tyr Val Lys Val Leu 13

78
Gln Glu Ser Ile Ser Phe Ser Leu Ser Pro Ser Lys Leu 26

117
Ala Cys Gln Lys Arg Pro Ser Gly Lys Leu Val Ser Val 39

156
Leu Ser Gly Ala Glu Gly Ser Phe Val Ser Ser Leu Val 52

195
Arg Ser Ile Ser Asn Ser Tyr Ser Tyr Ile Trp Ile Gly 65

234
Leu His Asp Pro Thr Gln Val Arg Val Tyr Pro Pro Leu 78

273
Ser Val Thr Ser Gln Gly Thr Val Val Ala Gln Ala His 91

306
Ser Leu Ser Pro Val Pro Ala Gln Glu Val Leu 102

15. Human PAP protein, characterized in that its amino acid sequence has a homology of at least 50%, preferably at least 60% and in very preferred manner at least 70% with at least one sequence of approximately 25 amino acids, included in the A2 sequence of the mature rat PAP protein or with the A3 sequence of the human PAP protein.

16. Amino acid sequence, characterized in that it is a fragment of the protein complying with the A3 amino acid sequence, said fragment being recognized by antibodies recognizing the A3 sequence.

17. Amino acid sequence according to claim 16, characterized in that it is recognized by monoclonal antibodies directed specifically against the A3 sequence.

18. Rat PAP protein, characterized in that it complies with the following amino acid sequence, or a variant or part of said sequence immediately said part or variant is recognized by antibodies directed against rat PAP protein, or has a homology of at least 50%, preferably at least 60% with the A2 sequence of rat PAP.

A1

MetLeuHisArgLeuAlaPheProValMetSerTrpMetLeuLeuLeuSerCysLeuMetLeuLeuSerGlnValGln

GlyGluAspSerProLysLysIleProSerAlaArgIleSerCysProLysGlySerGlnAlaTyrGlySerTyr

CysTyrAlaLeuPheGlnIleProGlnThrTrpPheAspAlaGluLeuAlaCysGlnLysArgProGluGlyHis

LeuValSerValLeuAsnValAlaGluAlaSerPheLeuAlaSerMetValLysAsnThrGlyAsnSerTyrGln

TyrThrTrpIleGlyLeuHisAspProThrLeuGlyGlyGluProAsnGlyGlyGlyTrpGluTrpSerAsnAsn

AspIleMetAsnTyrValAsnTrpGluArgAsnProSerThrAlaLeuAspArgGlyPheCysGlySerLeuSer

ArgSerGlyPheLeuArgTrpArgAspThrThrCysGluValGluValAlaLeuArgLeuGlnIleTyrArg

LeuLysLeuProAspSerLysGlnLeu

19. Antibodies, characterized in that they recognize human PAP protein according to any one of the claims 11 to 16 and in that they are polyclonal or monoclonal antibodies.

20. Monoclonal antibodies such as are obtained from a hybridoma, previously formed by the fusion of a myeloma cell and a splene cell of an animal previously immunized with a protein according to any one of the claims 11 to 16.

21. Monoclonal antibodies according to either of the claims 19 and 20, characterized in that they specifically recognize the terminal NH₂ part of the A3 sequence of amino acids.

22. Monoclonal antibodies according to either of the claims 19 and 20, characterized in that they are directed against the peptide Glu Glu Pro Gln Arg.

23. Hybridoma, such as is obtained from the fusion of a myeloma cell with a splene cell of an animal previously immunized with a human PAP protein according to any one of the claims 11 to 16.

24. Expression and/or cloning vector, characterized in that it comprises a DNA fragment according to any one of the claims 1 to 10.

25. Cellular host transformed by an expression vector according to claim 24, under conditions permitting the obtaining of the protein or peptide coded by the DNA fragment inserted in the vector, said cellular host being e.g. chosen from among bacteria, particularly in the case of E.coli from among yeasts, insect or mammal cells.

26. Expression product of the cellular host transformed according to claim 25.

27. Composition for the in vitro diagnosis of acute pancreatitis or for the observation of the pancreatic gland in medical imaging, in a biological sample such as blood, characterized in that it comprises at least one antibody according to any one of the claims 19 to 21, which, if appropriate, is labelled, and which is able to form a complex of the antibody-antigen type with the human PAP protein.

28. Kit for the in vitro diagnosis of acute pancreatitis from a biological sample, characterized in that it comprises at least one antibody according to any one of the claims 19 to 22 able to detect the presence of human PAP in said biological sample, said antibodies being, if appropriate, labelled, as a function of the nature of the labelling, a reagent for detecting the presence of a specific antibody-antigen type complex and a negative control.

29. Process for the in vitro detection of acute pancreatitis from a biological sample involving the stages of contacting the biological sample liable to contain human PAP with antibodies according to any one of the claims 19 to 22 and detection of the antigen-antibody reaction between said antibodies and the human PAP.

30. Kit according to claim 28 or process according to claim 29, characterized in that the antibodies are polyclonal antibodies.

31. Process for observing the pancreatic gland, characterized by the injection in a patient of a composition according to claim 27 under physiologically acceptable conditions and observation, in particular by medical imaging of a reaction between the antibodies contained in said composition and the human PAP, when present.

## Patentansprüche

1. Fragment von cDNA, das für ein PAP-Protein des Menschen charakteristisch ist und das man durch die folgenden Schritte erhalten kann ;
- erstes Durchmustern einer Genbank pankreatischer cDNA des Menschen, wobei die cDNA des Menschen in einen geeigneten Klonierungsvektor eingeführt wird und eine Hybridisierung mit Sonden stattfindet, die aus der cDNA des PAP-Proteins von Ratten bestehen, wie sie durch die folgenden Sequenzen S1 oder S2 dargestellt sind 
Sequenz S 1 :

10 20 30 40 50 60
AAAACCATCC AAATCGCCCG CAAGACAGCT AAGGAGGAGC AGAAAGATGA TGAGAGTTAA
70 80 90 100 110 120
TATGTTGCAT CGCTTGGCCT TCCCAGTCAT GTCCTGGATG CTGCTCTCCT GCCTGATGCT
130 140 150 160 170 180
CTTATCACAG GTGCAAGGAG AAGACTCTCC GAAGAAAATA CCCTCTGCAC GCATTAGTTG
190 200 210 220 230 240
CCCCAAAGGC TCCCAGGCAT ATGGCTCCTA CTGCTATGCC CTGTTTCAGA TACCACAGAC
250 260 270 280 290 300
CTGGTTTGAT GCAGAACTGG CCTGCCAGAA GAGACCTGAA GGACACCTTG TATCTGTGCT
310 320 330 340 350 360
CAATGTAGCT GAAGCTTCAT TCTTGGCATC CATGGTCAAG AACACTGGAA ACAGCTACCA
370 380 390 400 410 420
ATATACCTGG ATTGGACTCC ATGACCCCAC TCTTGGTGGA GAACCCAATG GAGGTGGATG
430 440 450 460 470 480
GGAGTGGAGT AACAATGACA TAATGAATTA TGTCAACTGG GAGAGGAACC CATCTACTGC
490 500 510 520 530 540
CTTAGACCGC GGATTCTGTG GCAGCTTGTC AAGATCTTCT GGATTTCTAA GATGGAGAGA
550 560 570 580 590 600
TACCACATGT GAAGTTGAAG TTGCCCTACG TCTGCAAATT TACAGGTTAA AATTACCAGA
610 620 630 640 650 660
CAGCAAACAG CTTTAGTTTG TCCTGAAGCA CATCCTGTCA AGGGGCAAAA TATGAAGACT
670 680 690 700 710 720
TGCGTAGAAA AAGTGTATTC TATCTACAGT CCATATTGGA GCTCTAATCA TTCTTTAGCC
730 740 750 760 770 780
AATTTTGTAT AAGTTGTGTC CTCATGTCTT GGAAAGCAGT AATAAACCTC AGTCTCTCTT
790 800 810 820 830 840
CGAAAAAAAA AAA
 
Sequenz S2 :

10 20 30 40 50 60
GAAGACT CTCCGAAGAA AATACCCTCT GCACGCATTA GTTGCCCCAA AGGCTCCCAG
70 80 90 100 110 120
GCATATGGCT CCTACTGCTA TGCCCTGTTT CAGATACCAC AGACCTGGTT TGATGCAGAA
130 140 150 160 170 180
CTGGCCTGCC AGAAGAGACC TGAAGGACAC CTTGTATCTG TGCTCAATGT AGCTGAAGCT
190 200 210 220 230 240
TCATTCTTGG CATCCATGGT CAAGAACACT GGAAACAGCT ACCAATATAC CTGGATTGGA
250 260 270 280 290 300
CTCCATGACC CCACTCTTGG TGGAGAACCC AATGGAGGTG GATGGGAGTG GAGTAACAAT
310 320 330 340 350 360
GACATAATGA ATTATGTCAA CTGGGAGAGG AACCCATCTA CTGCCTTAGA CCGCGGATTC
370 380 390 400 410 420
TGTGGCAGCT TGTCAAGATC TTCTGGATTT CTAAGATGGA GAGATACCAC ATGTGAAGTT
430 440 450 460 470 480
GAAGTTGCCC TACGTCTGCA AATTTACAGG TTAAAATTAC CAGACAGCAA ACAGCTT
 in einer Lösung, welche besteht aus: 6 x SSC, 5 x Denhart, 0,5 % SDS, 10 mM EDTA, 200 µg Lachssperma-DNA, über 18 Stunden hinweg bei 68 °C, mit nachfolgender Spülung unter den folgenden Bedingungen: 6 x SSC, 0,1 % SDS, 2mal 15 Minuten bei 65 °C,
- Auswählen der positiven cDNA-Klone des Menschen, welche während des Durchmuslerns mit der cDNA des PAP-Proteins von Ratten hybridisierten, wobei diese Klone als positiv bezeichnet werden,
- zweites Durchmustcrn mit einer cDNA-Sequenz eines PSP-Proteins ("Pancrealic Stone Protein") unter den obigen Hybridisierungsbedingungen mit Spülung in Gegenwart von 0,1 x SSC, 0,1 % SDS, über 2 Stunden hinweg bei 65 °C, um die nicht-spezifischen Klone der cDNA von Menschen-PAP zu entfernen, die dennoch mit der cDNA von Ratten-PAP hybridisierten, und
- Rückgewinnen der Klone, die nicht mit der PSP-cDNA hybridisierten,
- Rückgewinnen der cDNA-Fragmente der erhaltenen positiven Klone.

2. Fragment von cDNA, das für ein PAP-Protein des Menschen charakteristisch ist, nach Anspruch 1, dadurch gekennzeichnet, daß dem Schritt zum Durchmustern mit der PSP-cDNA ("Pancreatie Stone Protein") ein Schritt zum Durchmustern mit cDNA von Ratten-PAP folgt, wie sie durch die folgenden Sequenzen S1 oder S2 dargestellt sind 
Sequenz S 1 :

10 20 30 40 50 60
AAAACCATCC AAATCGCCCG CAAGACAGCT AAGGAGGAGC AGAAAGATGA TGAGAGTTAA
70 80 90 100 110 120
TATGTTGCAT CGCTTGGCCT TCCCAGTCAT GTCCTGGATG CTGCTCTCCT GCCTGATGCT
130 140 150 160 170 180
CTTATCACAG GTGCAAGGAG AAGACTCTCC GAAGAAAATA CCCTCTGCAC GCATTAGTTG
190 200 210 220 230 240
CCCCAAAGGC TCCCAGGCAT ATGGCTCCTA CTGCTATGCC CTGTTTCAGA TACCACAGAC
250 260 270 280 290 300
CTGGTTTGAT GCAGAACTGG CCTGCCAGAA GAGACCTGAA GGACACCTTG TATCTGTGCT
310 320 330 340 350 360
CAATGTAGCT GAAGCTTCAT TCTTGGCATC CATGGTCAAG AACACTGGAA ACAGCTACCA
370 380 390 400 410 420
ATATACCTGG ATTGGACTCC ATGACCCCAC TCTTGGTGGA GAACCCAATG GAGGTGGATG
430 440 450 460 470 480
GGAGTGGAGT AACAATGACA TAATGAATTA TGTCAACTGG GAGAGGAACC CATCTACTGC
490 500 510 520 530 540
CTTAGACCGC GGATTCTGTG GCAGCTTGTC AAGATCTTCT GGATTTCTAA GATGGAGAGA
550 560 570 580 590 600
TACCACATGT GAAGTTGAAG TTGCCCTACG TCTGCAAATT TACAGGTTAA AATTACCAGA
610 620 630 640 650 660
CAGCAAACAG CTTTAGTTTG TCCTGAAGCA CATCCTGTCA AGGGGCAAAA TATGAAGACT
670 680 690 700 710 720
TGCGTAGAAA AAGTGTATTC TATCTACAGT CCATATTGGA GCTCTAATCA TTCTTTAGCC
730 740 750 760 770 780
AATTTTGTAT AAGTTGTGTC CTCATGTCTT GGAAAGCAGT AATAAACCTC AGTCTCTCTT
790 800 810 820 830 840
CGAAAAAAAA AAA
 
Sequenz S2 :

10 20 30 40 50 60
GAAGACT CTCCGAAGAA AATACCCTCT GCACGCATTA GTTGCCCCAA AGGCTCCCAG
70 80 90 100 110 120
GCATATGGCT CCTACTGCTA TGCCCTGTTT CAGATACCAC AGACCTGGTT TGATGCAGAA
130 140 150 160 170 180
CTGGCCTGCC AGAAGAGACC TGAAGGACAC CTTGTATCTG TGCTCAATGT AGCTGAAGCT
190 200 210 220 230 240
TCATTCTTGG CATCCATGGT CAAGAACACT GGAAACAGCT ACCAATATAC CTGGATTGGA
250 260 270 280 290 300
CTCCATGACC CCACTCTTGG TGGAGAACCC AATGGAGGTG GATGGGAGTG GAGTAACAAT
310 320 330 340 350 360
GACATAATGA ATTATGTCAA CTGGGAGAGG AACCCATCTA CTGCCTTAGA CCGCGGATTC
370 380 390 400 410 420
TGTGGCAGCT TGTCAAGATC TTCTGGATTT CTAAGATGGA GAGATACCAC ATGTGAAGTT
430 440 450 460 470 480
GAAGTTGCCC TACGTCTGCA AATTTACAGG TTAAAATTAC CAGACAGCAA ACAGCTT
 in einer Hybridisierungslösung, welche 6 x SSC, 5 x Denhardt, 0,5 % SDS, 10 mM EDTA, 200 µg Lachssperma-DNA enthält, über 18 Stunden hinweg bei 68 °C, mit nachfolgender Spülung über 2 Stunden hinweg bei 65°C.

3. Fragment von cDNA von Menschen-PAP nach einem der Ansprüche 1 oder 2, dadurch gekennzeichnet, daß es der folgenden Sequenz S3 entspricht:

ATG CTG
CCT CCC ATG GCC CTG CCC AGT GTA TCT TGG ATG CTG CTT
TCC TGC CTC ATG CTG CTG TCT CAG GTT CAA GGT GAA GAA
CCC CAG AGG GAA CTG CCC TCT GCA CGG ATC CGC TGT CCC
AAA GGC TCC AAG GCC TAT GGC TCC CAC TGC TAT GCC TTG
TTT TTG TCA CCA AAA TCC TGG ACA GAT GCA GAT CTG GCC
TGC CAG AAG CGG CCC TCT GGA AAC CTG GTG TCT GTG CTC
AGT GGG GCT GAG GGA TCC TTC GTG TCC TCC CTG GTG AAG
AGC ATT GGT AAC AGC TAC TCA TAC GTC TGG ATT GGG CTC
CAT GAC CCC ACA CAG GGC ACC GAG CCC AAT GGA GAA GGT
TGG GAG TGG AGT AGC AGT GAT GTG ATG AAT TAC TTT GCA
TGG GAG AGA AAT CCC TCC ACC ATC TCA AGC CCC GGC CAC
TGT GCG AGC CTG TCG AGA AGC ACA GCA TTT CTG AGG TGG
AAA GAT TAT AAC TGT AAT GTG AGG TTA CCC TAT GTC TGC
AAA GTT CAC

4. Fragment von cDNA von Menschen-PAP nach einem der Ansprüche 1 oder 2, dadurch gekennzeichnet, daß es für die Kettenbildung der folgenden Aminosäuren A3 kodiert:

5. Fragment von cDNA, das für ein PAP-protein des Menschen charakteristisch ist, nach Anspruch 1 oder 2, dadurch gekennzeichnet, daß es eine Nukleotidsequenz aufweist, die eine Homologie von mindestens 60 %, vorzugsweise mindestens 70 %, mit mindestens einer Kette von etwa 100 Nukleotiden hat, die in der folgenden für cDNA von gereiftem Ratten-PAP charakteristischen Sequenz S2 oder in der für ein cDNA-Fragment von Menschen-PAP charakteristischen Sequenz S3 enthalten sind.

Sequenz S2 :

10 20 30 40 50 60
GAAGACT CTCCGAAGAA AATACCCTCT GCACGCATTA GTTGCCCCAA AGGCTCCCAG
70 80 90 100 110 120
GCATATGGCT CCTACTGCTA TGCCCTGTTT CAGATACCAC AGACCTGGTT TGATGCAGAA
130 140 150 160 170 180
CTGGCCTGCC AGAAGAGACC TGAAGGACAC CTTGTATCTG TGCTCAATGT AGCTGAAGCT
190 200 210 220 230 240
TCATTCTTGG CATCCATGGT CAAGAACACT GGAAACAGCT ACCAATATAC CTGGATTGGA
250 260 270 280 290 300
CTCCATGACC CCACTCTTGG TGGAGAACCC AATGGAGGTG GATGGGAGTG GAGTAACAAT
310 320 330 340 350 360
GACATAATGA ATTATGTCAA CTGGGAGAGG AACCCATCTA CTGCCCTTAGA CCGCGGATTC
370 380 390 400 410 420
TGTGGCAGCT TGTCAAGATC TTCTGGATTT CTAAGATGGA GAGATACCAC ATGTGAAGTT
430 440 450 460 470 480
GAAGTTGCCC TACGTCTGCA AATTTACAGG TTAAAAATTAC CAGACAGCAA ACAGCTT

6. Fragment von cDNA, das für ein PAP-Protein des Menschen charakteristisch ist, nach einem der Ansprüche 1 oder 2, dadurch gekennzeichnet, daß es in der Lage ist, zu hybridisieren mit der folgenden für die cDNA von Ratten-PAP charakteristischen Nukleotidsequenz S1 und/oder mit der für die cDNA von gereiftem Ratten-PAP charakteristischen Nukleotidsequenz S2 in einer Hybridisierungslösung, welche 6 x SSC, 5 x Denhart, 0,5 % SDS, 10 nM EDTA, 200 µg Lachssperma-DNA enthält, über 18 Stunden bei 68 °C hinweg und mit einer Spülung in einer Lösung, welche 6 x SSC, 0,1 % SDS enthält, 2mal während 15 Minuten bei 65 °C.

Sequenz S 1 :

10 20 30 40 50 60
AAAACCATCC AAATCGCCCG CAAGACAGCT AAGGAGGAGC AGAAAGATGA TGAGAGTTAA
70 80 90 100 110 120
TATGTTGCAT CGCTTGGCCT TCCCAGTCAT GTCCTGGATG CTGCTCTCCT GCCTGATGCT
130 140 150 160 170 180
CTTATCACAG GTGCAAGGAG AAGACTCTCC GAAGAAAATA CCCTCTGCAC GCATTAGTTG
190 200 210 220 230 240
CCCCAAAGGC TCCCAGGCAT ATGGCTCCTA CTGCTATGCC CTGTTTCAGA TACCACACAC
250 260 270 280 290 300
CTGGTTTGAT GCAGAACTGG CCTGCCAGAA GAGACCTGAA GGACACCTTG TATCTGTGCT
310 320 330 340 350 360
CAATGTAGCT GAAGCTTCAT TCTTGGCATC CATGGTCAAG AACACTGGAA ACAGCTACCA
370 380 390 400 410 420
ATATACCTGG ATTGGACTCC ATGACCCCAC TCTTGGTGGA GAACCCAATG GAGGTGGATG
430 440 450 460 470 480
GGAGTGGAGT AACAATGACA TAATGAATTA TGTCAACTGG GAGAGGAACC CATCTACTGC
490 500 510 520 530 540
CTTAGACCGC GGATTCTGTG GCAGCTTGTC AAGATCTTCT GGATTTCTAA GATGGAGAGA
550 560 570 580 590 600
TACCACATGT GAAGTTGAAG TTGCCCTACG TCTGCAAATT TACAGGTTAA AATTACCAGA
610 620 630 640 650 660
CAGCAAACAG CTTTAGTTTG TCCTGAAGCA CATCCTGTCA AGGGGCAAAA TATGAAGACT
670 680 690 700 710 720
TGCCGTAGAA AAGTGTATTC TATCTACAGT CCATATTGGA GCTCTAATCA TTCTTTAGCC
730 740 750 760 770 780
AATTTTGTAT AAGTTGTGTC CTCATGTCTT GGAAAGCAGT AATAAACCTC AGTCTCTCTT
790 800 810 820 830 840
CGAAAAAAAA AAA

7. Fragment von cDNA, die für ein PAP-Protein des Menschen charakteristisch ist, nach Anspruch 1 oder 2, dadurch gekennzeichnet, daß es die folgende Nukleolidsequenz aufweist:

TTTGTTAAGG ATTCCCTTGA GAATTATGTA AAAGTTTTAC AAGAGTCCAT CTCATTCTCT
TTGTCCCCCT CAAAGCTGGC TTGCCAGAAG CGGCCCTCTG GAAAACTGGT GTCTGTGCTC
AGTGGGCCTG AGGGATCCTT CGTGTCCTCC CTGGTGAGGA GCATTAGTAA CAGCTACTCA
TACATCTGGA TTGGGCTCCA TGACCCCACA CAGGTGCCAG TATATCCTCC CCTCTCTGTT
ACCTCTCAAG GTACTGTTGT TGCCCAGGCG CACTCCCTGT CCCCAGTCCC TGCCCAGGAA
GTACTT

8. Fragment von cDNA, dadurch gekennzeichnet, daß es der Nukleotidsequenz S1 entspricht, die für das PAP-Protein von Ratten kodiert, oder einem Teil oder einer Variante dieser Sequenz, sobald dieser Teil oder diese Variante für ein Protein oder ein Peptid kodiert, das von Antikörpern erkannt wird, die gegen das PAP-Protein von Ratten gerichtet sind, oder daß es mit der Sequenz S1 in einer Hybridisierungslösung hybridisiert, die 6 x SSC, 5 x Denhart, 0,5 % SDS, 10 mM EDTA, 200 µg Lachssperma-DNA enthält, und zwar 18 Stunden bei 68 °C und nach einem Spülen unter den folgenden Bedingungen: 6 x SSC, 0,1 % SDS, 2mal 15 Minuten bei 65 °C.

9. Fragment von cDNA, dadurch gekennzeichnet, daß es für ein Protein kodiert, das einer der folgenden Aminosäure-Sequenzen A1 oder A2 entspricht, oder für eine Aminosäure-Sequenz kodiert, die eine Homologie von 40 bis 80 %, vorzugsweise 50 bis 60%, mit mindestens einer Kette von etwa 25 Aminosäuren der Sequenzen A1 oder A2 hat, oder in der Sequenz A3 des PAP-Proteins des Menschen.

A1

MetLeuHisArgLeuAlaPheProValMetSerTrpMetLeuLeuLeuSerCysLeuMetLeuLeuSerGlnValGln

GlyGluAspSerProLysLysIleProSerAlaArgIleSerCysProLysGlySerGlnAlaTyrGlySerTyr

CysTyrAlaLeuPheGlnIleProGlnThrTrpPheAspAlaGluLeuAlaCysGlnLysArgProGluGlyHis

LeuValSerValLeuAsnValAlaGluAlaSerPheLeuAlaSerMetValLysAsnThrGlyAsnSerTyrGln

TyrThrTrpIleGlyLeuHisAspProThrLeuGlyGlyGluProAsnGlyGlyGlyTrpGluTrpSerAsnAsn

AspIleMetAsnTyrValAsnTrpGluArgAsnProSerThrAlaLeuAspArgGlyPheCysGlySerLeuSer

ArgSerSerGlyPheLeuArgTrpArgAspThrThrCysGluValGluValAlaLeuArgLeuGlnIleTyrArg

LeuLysLeuProAspSerLysGlnLeu

λ2

GluAspSerProLysLysIleProSerAlaArgIleSerCysProLysGlySerGlnAlaTyrGlySerTyr

CysTyrAlaLeuPheGlnIleProGlnThrTrpPheAspAlaGluLeuAlaCysGlnLysArgProGluGlyHis

LeuValSerValLeuAsnValAlaGluAlaSerPheLeuAlaSerMetValLysAsnThrGlyAsnSerTyrGln

TyrThrTrpIleGlyLeuHisAspProThrLeuGlyGlyGluProAsnGlyGlyGlyTrpGluTrpSerAsnAsn

AspIleMetAsnTyrValAsnTrpGluArgAsnProSerThrAlaLeuAspArgGlyPheCysGlySerLeuSer

ArgSerSerGlyPheLeuArgTrpArgAspThrThrCysGluValGluValAlaLeuArgLeuGlnIleTyrArg

LeuLysLeuProAspSerLysGlnLeu

10. Nukleinsäure-Fragment, dadurch gekennzeichnet, daß es sich um ein komplementäres DNA-Fragment der cDNA nach einem der Ansprüchc 1 bis 9 handelt oder daß es sich um das dieser cDNA entsprechende RNA-Fragment handelt.

11. PAP-Protein des Menschen, wie man es durch die Expression eines cDNA-Fragments von Menschen-PAP erhält, wie man es nach Anspruch 1 oder 2 mittels eines angepaßten Expressionssystems erhält, wie z.B. einem mittels eines Expressionsvektors transformierten Zellwirt, der seinerseits durch Einfügen des obigen cDNA-Fragmcnts der Menschen-PAP modifiziert wurde.

12. PAP-Protein des Menschen nach Anspruch 11, wie es mittels der Expression eines cDNA-Fragments von Menschen-PAP erhalten wird, das phasengleich in einen pEX-Vektor eingeführt wird, und zwar in E.coli, insbesondere in E.coli Stamm pop2136.

13. PAP-Protein des Menschen nach einem der Ansprüche 1 bis 12, dadurch gekennzeichnet, daß es der Aminosäurekette A3 entspricht.

14. PAP-Protein des Menschen nach Anspruch 11 oder 12, dadurch gekennzeichnet, daß es die folgende Aminosäurekette aufweist:

39
Phe Val Lys Asp Ser Leu Glu Asn Tyr Val Lys Val Leu 13

78
Gln Glu Ser Ile Ser Phe Ser Leu Ser Pro Ser Lys Leu 26

117
Ala Cys Gln Lys Arg Pro Ser Gly Lys Leu Val Ser Val 39

156
Leu Ser Gly Ala Glu Gly Ser Phe Val Ser Ser Leu Val 52

195
Arg Ser Ile Ser Asn Ser Tyr Ser Tyr Ile Trp Ile Gly 65

234
Leu His Asp Pro Thr Gln Val Arg Val Tyr Pro Pro Leu 78

273
Ser Val Thr Ser Gln Gly Thr Val Val Ala Gln Ala His 91

306
Ser Leu Ser Pro Val Pro Ala Gln Glu Val Leu 102

15. PAP-Protein des Menschen, dadurch gekennzeichnet, daß seine Aminosäure-Sequenz eine Homologie von mindestens 50 %, vorzugsweise mindestens 60 % und besonders bevorzugt mindestens 70 % mit mindestens einer Kette von etwa 25 Aminosäuren, die in der Sequenz A2 des gereiften PAP-Proteins von Ratten enthalten ist, oder mit der Sequenz A3 des PAP-Proteins des Menschen aufweist.

16. Aminosäure-Sequenz, dadurch gekennzeichnet, daß es sich um ein Fragment des Proteins handelt, das der Aminosäure-Sequenz A3 entspricht, wobei das Fragment von Antikörpern erkannt wird, welche die Sequenz A3 erkennen.

17. Aminisäure-Sequenz nach Anspruch 16, dadurch gekennzeichnet, daß sie von monoklonalen Antikörpern erkannt wird, die spezifisch gegen die Sequenz A3 gerichtet sind.

18. PAP-Protein von Ratten, dadurch gekennzeichnet, daß es der folgenden Aminosäurekette oder einer Variante oder einem Teil dieser Kette entspricht, sobald dieser Teil oder diese Variante durch Antikörper erkannt wird, die gegen das PAP-Protein von Ratten gerichtet sind, oder daß es eine Homologie von mindestens 50 %, vorzugsweise mindestens 60 % mit der Sequenz A2 von Ratten-PAP aufweist.

A1

MetLeuHisArgLeuAlaPheProValMetSerTrpMetLeuLeuSerCysLeuMetLeuLeuSerGlnValGln

GlyGluAspSerProLysLysIleProSerAlaArgIleSerCysProLysGlySerGlnAlaTyrGlySerTyr

CysTyrAlaLeuPheGlnIleProGlnThrTrpPheAspAlaGluLeuAlaCysGlnLysArgProGluGlyHis

LeuValSerValLeuAsnValAlaGluAlaSerPheLeuAlaSerMetValLysAsnThrGlyAsnSerTyrGln

TyrThrTrpIleGlyLeuHisAspProThrLeuGlyGlyGluProAsnGlyGlyGlyTrpGluTrpSerAsnAsn

AspIleMetAsnTyrValAsnTrpGluArgAsnProSerThrAlaLeuAspArgGlyPheCysGlySerLeuSer

ArgSerSerGlyPheLeuArgTrpArgAspThrThrCysGluValGluValAlaLeuArgLeuGlnIleTyrArg

LeuLysLeuProAspSerLysGlnLeu

19. Antikörper, dadurch gekennzeichnet, daß sie das PAP-Protein des Menschen nach einem der Ansprüche 11 bis 16 erkennen und daß es sich um polyklonale oder monoklonale Antikörper handelt.

20. Monoklonale Antikörper, wie sie ausgehend von einem Hybridom gewonnen werden, das vorher durch Verschmelzung einer Myelom-Zelle und einer splenischen Zelle eines Tiers, das zuvor mit einem Protein nach einem der Ansprüche 11 bis 16 immunisiert wurde, gebildet wurde.

21. Monoklonale Antikörper nach einem der Ansprüche 19 oder 20, dadurch gekennzeichnet, daß sie spezifisch den NH₂-terminalen Teil der Aminosäure-Kette A3 erkennen.

22. Monoklonale Antikörper nach einem der Ansprüche 19 oder 20, dadurch gekennzeichnet, daß sie gegen das Peptid Glu Glu Pro Gln Arg gerichtet sind.

23. Hybridom, wie es ausgehend von der Verschmelzung einer Myelom-Zelle mit einer splenischen Zelle eines Tiers, das zuvor mit einem PAP-Protein des Menschen nach einem der Ansprüche 11 bis 16 immunisiert wurde, gewonnen wird.

24. Expressions- und/oder Klonierungsvektor, dadurch gekennzeichnet, daß er ein DNA-Fragment nach einem der Ansprüche 1 bis 10 aufweist.

25. Zellwirt, der mittels eines Expressionsvektors nach Anspruch 24 unter Bedingungen transformiert wurde, welche die Gewinnung des Proleins oder des Peptids ermöglichen, das durch das in den Vektor eingeführte DNA-Fragment kodiert wird, wobei der Zellwirt z.B. aus Bakterien, insbesondere E.coli, aus Hefen, Insekten- oder Säugetierzellen ausgewählt wird.

26. Expressionsprodukt des nach Anspruch 25 transformierten Zellwirls.

27. Zusammensetzung für die in vitro-Diagnose akuter Pankreatitis oder für die Beobachtung der Bauchspeicheldrüse durch medizinische Bilderfassung in einer biologischen Probe, wie z.B. Blut, dadurch gekennzeichnet, daß sie mindestens einen, gegebenenfalls markierten Antikörper nach einem der Ansprüche 19 bis 21 enthält, der in der Lage ist, einen Antigen-Antikörper-Komplex mit dem PAP-Protein des Mensehen zu bilden.

28. Kit für die in vitro-Diagnose akuter Pankreatitis ausgehend von einer biologischen Probe, dadurch gekennzeichnet, daß es aufweist:
- mindestens einen Antikörper nach einem der Ansprüche 19 bis 22, der in der Lage ist, das Vorhandensein von Menschen-PAP in der biologischen Probe zu detektieren, wobei die Antikörper gegebenenfalls markiert sind,
- je nach Markierung ein Reagenz zum Detektieren des Vorhandenseins eines spezifischen Antigen-Antikörper-Komplexes,
- einen Negativ-Anzeiger.

29. Verfahren zur in vitro-Erfassung einer akuten Pankrealitis, ausgehend von einer biologischen Probe, mit den folgenden Schritten:
- Zusammenbringen der biologischen Probe, welche das Menschen-PAP enthalten kann, mit Antikörpern nach einem der Ansprüche 19 bis 22,
- Detektion der Antigen-Antikörper-Reaktion zwischen den obengenannten Antikörpern und dem Menschen-PAP.

30. Kit nach Anspruch 28 oder Verfahren nach Anspruch 29, dadurch gekennzeichnet, daß die Antikörper polyklonale Antikörper sind.

31. Verfahren zur Beobachtung der Bauchspeicheldrüse, gekennzeichnet durch:
- Injizieren einer Zusammensetzung nach Anspruch 27 in einen Patienten unter physiologisch akzeptablen Bedingungen,
- Beobachten, insbesondere mittels medizinischer Abbildungstechniken, einer Reaktion zwischen den in der obengenannten Zusammensetzung enthaltenen Antikörpern und dem Menschen-PAP, wenn es vorhanden ist.
